# EUROPEAN PATENT SPECIFICATION

(11) **EP 3 818 935 B1**
(45) Date of publication and mention of the grant of the patent: **26.11.2025**
(21) Application number: 20203080.5
(22) Date of filing: 21.10.2020
(51) Int. Cl.: A61B 5/145, A61B 5/00

(54) **TESTING COGNITIVE ABILITY OF USER TO SELF-TREAT FROM MEDICAL EVENT**
PRÜFUNG DER KOGNITIVEN FÄHIGKEIT DES BENUTZERS, SICH NACH EINEM MEDIZINISCHEN EREIGNIS SELBST ZU BEHANDELN
TEST DE LA CAPACITÉ COGNITIVE DE L'UTILISATEUR À S'AUTO-SOIGNER À LA SUITE D'UN ÉVÉNEMENT MÉDICAL

(30) Priority: 22.10.2019 US 201916660698
(43) Date of publication of application: 12.05.2021
(73) Proprietor: Roche Diabetes Care GmbH, 68305 Mannheim (DE); F. Hoffmann-La Roche AG, 4070 Basel (CH)
(72) Inventor: MEARS, Mark G, Indianapolis, Indiana 46250 (US)
(74) Representative: J A Kemp LLP

(56) References cited:
- US-A1- 2011 163 881
- US-B2- 10 052 073

## Description

### BACKGROUND

A person with diabetes may be alerted by a blood glucose monitoring device to current or future predicted out-of-normal-glucose-range events which may be imminently dangerous, such as hyperglycemia or hypoglycemia. During these out-of-normal-glucose-range events, the user may have such elevated mental confusion that that the user fails to realize the dangerous state is occurring and/or take action to treat their glucose state. A user's phone or continuous glucose monitor (CGM) controller device may be an external device to a user that may alert the user of the imminently dangerous event, but the user may not be near the phone or CGM controller device. Some systems may also contact an emergency service when the user is initially unresponsive to such alerts.

The user's lack of response, however, may not indicate that the user is in need of emergency assistance. It may be undesirable to contact an emergency service when user is capable of self-treatment. Communicating emergency alerts or contacting emergency services when a user is aware of their diabetic state and capable of self-treatment may waste communication resources (*e.g*., communicating to emergency contacts, communicating messages over a network unnecessarily, *etc.*) and processing resources on a device providing alerts.

Some insulin pumps are also now integrated with systems for automatically controlling the insulin levels in response to changes in blood glucose levels. For example, insulin pumps are integrated with CGMs and can shut off insulin delivery when blood glucose levels are dropping too quickly to help prevent hypoglycemia. If the user is aware of the diabetic event and capable of self-treatment, the resources on the insulin pump may also be preserved when the user implements another form of treatment.

US 2011/0163881 Al discloses system and method responsive to an event detected at a glucose monitoring device. Other relevant prior art is disclosed in US10052073 B2.

### SUMMARY

The invention is defined in the independent claims. A user may be alerted by one or more user devices (*e.g.,* a mobile device, a CGM controller, a BGM, and/or another device) when the blood glucose level of the user is above a predefined threshold or below a predefined threshold that indicate the user has reached, or may imminently reach, an extreme diabetic state. The predefined threshold may be a high-end threshold that indicates that the user has reached, or may imminently reach, a hyperglycemic state. The predefined threshold may be a low-end threshold that indicates that the user has reached, or may imminently reach, a hypoglycemic state. When the predefined threshold for the blood glucose level of the user is reached, the user be in a state of elevated mental confusion and may be unable to realize the extreme state or take immediate action to self-treat the diabetic condition.

The user may be provided with a self-treat test that includes pass/fail criteria to test the ability of the user to perform self-treatment. The self-treat test may be provided by one or more user devices, such as the mobile device, the CGM controller, the BGM, and/or the insulin pump. The self-treat test may be initially performed by a user to set a baseline when the user's blood glucose is at a normal level. The same test that has been previously provided to the user to set the baseline may be provided to determine whether the user is capable of performing self-treatment when the user's blood glucose levels have increased or decreased.

The self-treat test may comprise a cognitive test, such as a concentration test, a math test, or a memory test. In the cognitive test, the pass/fail criteria may include at least one question to the user. The self-treat test may test the dexterity of the user. For example, the pass/fail criteria may comprise a relative change in acceleration or a relative change in orientation of the user's mobile device over a period of time, an ability of the user to trace a predefined shape on a display of a user device, and/or an ability of the user to identify a predefined sequence of shapes displayed on a screen to the user.

A timer may be set to give the user time to perform the self-treat test and/or treat the extreme diabetic state. The timer may be set differently based on different levels of severity for the extreme diabetic state. A longer timer may be triggered after detection of a lower level of severity associated with the extreme diabetic state than for higher levels of severity. The timer may allow a user time to treat the extreme diabetic state and/or provide information indicating that treatment has occurred. As different users may react differently to different blood glucose levels, the timer may be user-defined or defined by a third party (*e.g*., such as a physician) on the user device and stored in memory.

If the user passes the self-treat test, the user may be assumed to be able to treat the diabetic condition themselves. If the user fails the test, a diabetes-related alert may be provided via a user device. The diabetes-related alert may be an audible alert communicated via a speaker of the mobile device. The diabetes-related alert may be communicated otherwise, for example, to an external speaker device for audible communication to an occupant of a space, via telecommunication or text message, and/or via a home automation system.

### BRIEF DESCRIPTION OF THE DRAWINGS

FIG. 1 is a perspective view of a representative environment for monitoring and/or treating a diabetic condition.
FIG. 2 is a flow diagram of an example method for providing a self-treat test to a user to generate a baseline ability of the user to perform self-treatment of a diabetic condition.
FIG. 3A is a flow diagram of an example method for providing a self-treat test to a user to test the ability of the user to perform self-treatment.
FIG. 3B is another flow diagram of an example method for providing a self-treat test to a user to test the ability of the user to perform self-treatment.
FIGs. 4A-4D depicts example graphical user interfaces (GUIs) that may be displayed on a user device.
FIG. 5 is a block diagram of an example computing device.
FIG. 6 is a block diagram of an example blood glucose monitoring device.
FIG. 7 is a block diagram of an example blood glucose measuring (BGM) device.
FIG. 8 is a block diagram illustrating an example of an insulin pump.

### DETAILED DESCRIPTION

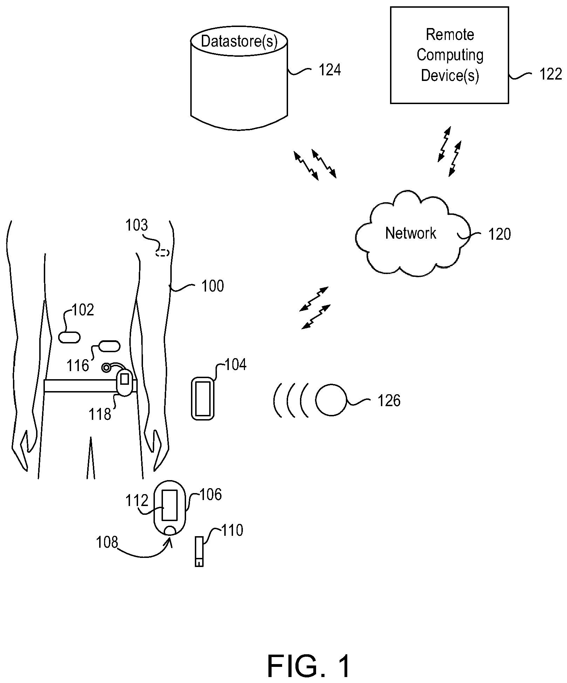

FIG. 1 is a perspective view of a representative environment for monitoring and/or treating a diabetic condition. As shown in FIG. 1, a user 100 with diabetes may use one or more devices to help monitor and/or treat the diabetic condition. The diabetic condition may include a metabolic syndrome, pre-diabetes, type 1 diabetes, type 2 diabetes, and/or gestational diabetes. The user 100 may be in an extreme diabetic state, such as hypoglycemia or hyperglycemia, when the blood glucose level of the user 100 is above or below a threshold blood glucose level. The user 100 may use a blood glucose monitoring device to monitor blood glucose levels.

The blood glucose monitoring device may be a continuous glucose monitor (CGM) 102. The CGM 102 may include a subcutaneous sensor that is used to sense and monitor the amount of glucose in interstitial fluid of the user 100. The CGM 102 may include a transmitting device that is located directly over the sensor that wirelessly powers the data transfer from the sensor. The CGM 102 may periodically communicate data indicating the blood glucose levels of the user 100 to an external device, such as a mobile device 104, for computing and/or storing the blood glucose levels of the user 100. The mobile device 104 may operate as a CGM controller device. Though the mobile device 104 may be provided as an example of a device with which the CGM 102 may communicate, the CGM 102 may communicate with other dedicated CGM controller devices for providing similar functionality that is described herein for the mobile device 104. The mobile device 104, or another CGM controller device, may provide audible alerts, graphical user interfaces, or non-audible alerts in response to data indications or triggers identified in the monitored blood glucose data. The CGM 102 may include an electric motor for providing on-body vibration alerts and/or a speaker for providing audible alerts in response to data indications or triggers identified in the monitored blood glucose data. The CGM 102 may process the blood glucose data for providing alerts, or the blood glucose data may be processed at the mobile device 104, or other CMG controller device, an alert indicator may be communicated to the CGM 102.

The blood glucose monitoring may be performed by flash glucose monitoring (FGM). The FGM may include a subcutaneous sensor 103 that is used to sense and monitor the amount of glucose in interstitial fluid of the user 100. A separate reader device, such as the mobile device 102 or another reader device, may receive the blood glucose information from the sensor when the device is within the RF range of the sensor 103. The sensor 103 may transmit an instantaneous blood glucose level and/or a graphical trend of the blood glucose level to the reader device for display.

The user 100 may use a blood glucose meter (BGM) 106 as a blood glucose monitoring device to monitor blood glucose levels. The BGM 106 may include port 108 that may receive a blood glucose measurement strip 110. The user 100 may deposit a sample of blood on the blood glucose measurement strip 110. The BGM 106 may analyze the sample and measure the blood glucose level in the sample. The blood glucose level measured from the sample may be displayed on a display 112 of the BGM 106 and/or communicated to an external device, such as the mobile device 104.

The blood glucose level measured by the BGM 106 and/or computed using data received from the CGM 102 may be used to treat the diabetic condition of the user 100. For example, the user 100 may use an ambulatory non-durable insulin pump 116 or an ambulatory durable insulin pump 118 to treat the diabetic condition with insulin. The mobile device 104 may determine an amount of insulin to be administered to the user 100 and the insulin pump 116, 118 may receive instructions from the mobile device 104 to deliver a predetermined amount of insulin to the user 100. The insulin pump 116, 118 may receive other information from the mobile device 104, such as mealtime information and/or exercise information of the user 100. The insulin pump 116, 118 may determine the amount of insulin to administer based on the received information from the mobile device 104. The insulin pump 116, 118 may communicate information to the mobile device 104. The information communicated to the mobile device 104 may include an amount of insulin delivered to the user 100, corresponding times of delivery, and/or a pump status (*e.g.,* battery status, insulin status, *etc.*).

The mobile device 104 may communicate with the insulin pump 116, 118, the CGM 102, and/or the BGM 106 using wired and/or wireless communications. The mobile device 104, the CGM 102, a CGM controller, the BGM 106, and/or the insulin pump 116, 118 may be collectively referred to as user devices. The mobile device 104 may communicate with the other user devices using the same or different wireless protocols. For example, the mobile device 104 may communicate with the other user devices using BLUETOOTH^{®}, near field communication (NFC), THREAD^{®}, WIFI^{®}, ZIGBEE^{®}, WI-MAX^{®}, a cellular communication protocol, a proprietary wireless communication protocol, or another radio frequency (RF) communication protocol.

The mobile device 104 may be a mobile phone, a tablet, a laptop, a wearable device, a personal digital assistant (PDA), or another mobile computing device. If the mobile device 104 is a wearable device, the wearable device may be an armband (*e.g.,* a smart watch, such as an APPLE^{®} watch, a FITBIT^{®} armband, or other device capable of being worn on the arm of the user 100), a ring, glasses (*e.g.,* GOOGLE^{®} GLASS^{™}), a headset (*e.g.*, BLUETOOTH^{®} headset), clothing (*e.g.,* shirts, gloves, *etc.*), or another wearable device capable of being worn by the user 100. Though a mobile device may be described herein, other computing devices may be similarly implemented to perform functions of the mobile device.

The mobile device 104 may receive data and store data for assisting in monitoring and/or treating the diabetic condition. The mobile device 104 may receive input from the user 104 via a user interface being provided on a display. The mobile device 104 may receive input via hard buttons or soft buttons provided on the display.

The mobile device 104 may be configured to determine the device's location. For example, the mobile device 104 may be able to determine the geolocation (*e.g*., latitude and longitude) of the device using signals from a global positioning system (GPS) or triangulation via cellular communications. The mobile device 104 may determine a relative location using an RF beacon device 126. The RF beacon device 126 may communicate a unique identifier via a short-range wireless communication, such as a BLUETOOTH^{®} low energy (BLE) beacon or an NFC beacon. The mobile device 104 may receive the RF beacon and perform a lookup in a database (*e.g*., in information from the datastores 124) to determine a relative location associated with the unique identifier. For example, the mobile device 104 may determine that the RF beacon indicates that the device is in a particular room in a home or building, on a certain floor in a building, close to a predefined object, or is within the RF range of a beacon associated with another object or location.

The mobile device 104 may include one or more sensors for detecting a relative position of the device or information about the user 100. The mobile device 104 may detect a movement or a change in orientation. Based on the movement and/or change in orientation (or lack thereof) of the mobile device 104 over a period of time, the mobile device 104 may detect that the user 100 is standing, sitting, or lying down. The mobile device 104 may detect that the user 100 is exercising when the movement and/or the change in orientation is greater than a threshold for a period of time. The mobile device 104 may detect the heartrate of the user 100 using a heartrate sensor. Based on the heartrate and the movement of the user 100 over a period of time, the mobile device 104 may detect whether the user 100 is asleep or awake. The information about the mobile device 104 and/or the user 100 may be used to provide information about and/or treat the diabetic condition.

The mobile device 104 may provide information to the user 100 about the user's diabetic condition. For example, the mobile device 104 may provide blood glucose levels, provide meal-related information, provide exercise-related information, generate graphs and other graphical user interfaces for display, and/or generate alerts that may be provided to the user 100. For example, the mobile device 104 may measure the blood glucose level of the user 100 and provide an alert when the blood glucose level of the user 100 has reached a threshold for an extreme diabetic state (*e.g*., hypoglycemia or hyperglycemia). The alerts provided by the mobile device 104 may be audible and/or non-audible alerts. The non-audible alerts may be provided as a vibration, a flashing of the screen, or a flashing of an LED on the mobile device 104. The alerts may also, or alternatively, be provided by an external device based on a communication from the mobile device 104.

The mobile device 104 may communicate with other devices directly via a wired communication or a short-range wireless communication (*e.g*., WI-FI^{®}, BLUETOOTH^{®}, BLE, NFC, *etc.*). The mobile device 104 may communicate indirectly with remote computing devices 122 and/or datastores 124 via a network 120 (*e.g.,* using a WI-FI^{®} network, a cellular network, a WI-MAX^{®} network, *etc.*). The network 120 may be a wired and/or wireless network. The network 120 may be used to communicate over the Internet to other devices.

The mobile device 104 may communicate with the remote computing devices 122 to generate user interfaces for display on the mobile device 104, perform remote computation, and/or otherwise control a remote computing device. For example, the mobile device 104 may provide a user interface via an application or web browser that is generated at a remote computing device 122. The mobile device 104 may generate instructions for providing alerts via remote computing devices 122 based on information received from the user 100, the CGM 102, the BGM 106, and/or the insulin pump 116, 118. Example remote computing devices 122 to which the mobile device 104 may send communications for performing alerts may include a remote computer (*e.g.,* a server, a laptop, or other computer), an external speaker, an external display device (*e.g.,* television, monitor, *etc.*), a home automation system, remote telecommunications devices (*e.g.,* for sending text or voice communications to an emergency contact over a telecommunications network), or another remote computing device.

The mobile device 104 may communicate with the datastores 124 to store information and/or retrieve information. The information may include information related to the user 100, the CGM 102, the BGM 106, and/or the insulin pump 116, 118. For example, the mobile device 104 may receive treatment information associated with the user 100 as input or receive blood glucose information from the CGM 102 or the BGM 106 and send the information to the datastores 124 via the network 120. Stored information may be retrieved from the datastore 124 for treatment of the diabetic condition of the user. For example, the mobile device 104 may retrieve an amount of insulin delivered to the user 100 and/or corresponding times of delivery. The datastores 124 may include one or more remote storage locations, which may be collectively referred to as cloud storage.

As described herein, the user 100 may be alerted by one or more devices (*e.g.,* the mobile device 104, the CGM 102, a CGM controller, the BGM 106, and/or another device) when the blood glucose level of the user 100 is above a predefined threshold or below a predefined threshold that indicate the user has reached, or may imminently reach, an extreme diabetic state. The predefined threshold may be a high-end threshold that indicates that the user 100 has reached, or may imminently reach, a hyperglycemic state. The predefined threshold may be a low-end threshold that indicates that the user 100 has reached, or may imminently reach, a hypoglycemic state. When the predefined threshold for the blood glucose level of the user 100 is reached, the user 100 may be in a state of elevated mental confusion and may be unable to realize the extreme state or take immediate action to self-treat the diabetic condition.

The user 100 may be provided with a self-treat test that includes pass/fail criteria to test the ability of the user 100 to treat themselves. The self-treat test may be provided by one or more user devices, such as the mobile device 104, the CGM 102, a CGM controller device, the BGM 106, and/or the insulin pump 116, 118. The self-treat test may be generated at a remote computing device 122 and/or information in the test may be retrieved from a datastore 124, and the test may be provided via one or more of the user devices. The self-treat test may be the same test that has been previously provided to the user 100 when the user 100 is determined to be in a state in which the user 100 is capable of self-treatment. For example, the self-treat test may be initially administered to the user 100 when the user 100 is determined to be in a normoglycemic state from the blood glucose level of the user 100 to generate a baseline from which a subsequent self-treat test may be compared.

The self-treat test may be implemented to users that may be symptomatic or asymptomatic. The self-treat test may assist symptomatic and/or asymptomatic users in detecting an extreme diabetic state earlier than may be otherwise detected. For example, an asymptomatic user may fail to detect the extreme diabetic state due to the user's failure to be alerted by symptoms that may be detectable by a symptomatic user. In a symptomatic user, the symptoms may be delayed or may fail to be detected by the user early enough to allow for proper treatment and/or assistance to be solicited.

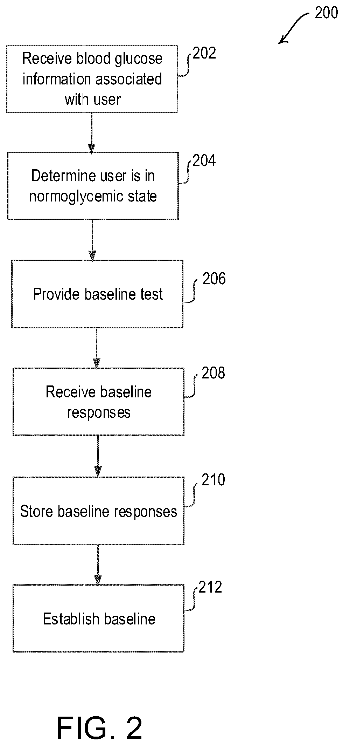

FIG. 2 is a flow diagram of an example method 200 for providing a self-treat test to a user to generate a baseline ability of the user to perform self-treatment of a diabetic condition. The method 200 may be performed my one or more computing devices. For example, the method 200 may be performed by a user device, such as the mobile device, the CGM, the BGM, or the insulin pump, or may be distributed across multiple user devices. Though an individual device may be described as performing one or more portions of the method 200, the portions of the method 200 may be performed on another device. For example, though portions of the method 200 may be performed at a user device, the portions may be performed at a remote device and resulting information may be received by the user device on which other portions of the method may be performed.

As shown in FIG. 2, blood glucose information may be received at 202. The blood glucose information may be detected by a CGM or a BGM and may be transmitted to another user device. For example, the blood glucose information may be received, via a communication circuit, at a mobile device or another user device. The blood glucose information may include the blood glucose levels measured by the BGM or data from the CGM indicating glucose levels of the user. The blood glucose level of the user may be generated from the CGM data. A determination may be made by a processor of a user device, at 204, that the user is in a stable, or normoglycemic, state. The normoglycemic state may be detected by determining that the blood glucose level of the user is less than a predefined high-end threshold and/or greater than a predefined low-end threshold.

A baseline test may be provided to the user at 206 to test the baseline ability of the user to perform self-treatment of a diabetic condition when the user is in the normoglycemic state. The baseline test may be provided by the processor of the user device, such as the user's mobile device. For example, the baseline test may be provided during setup of an application on the user's mobile device. The baseline test may include predefined pass/fail criteria for testing the user's ability to treat the diabetic condition themselves.

The baseline test may be a cognitive test that may test the mental state of the user by testing the user's ability cognitive ability to infer the user's ability to perform self-treatment. For example, the test may be a memory test that tests the ability to retain and recall information. The pass/fail criteria for the memory test may include an ability to answer generally well-known trivia questions to test the recall of the user. The trivia test may ask the user "Who is the current president of the United States?". The pass/fail criteria for the memory test may include an ability to recall an object that is flashed on the screen of the user device, such as a number, letter, or shape.

The test may be a concentration test that may test the mental state of the user by testing the user's ability to concentrate. The pass/fail criteria for the concentration test may include an ability to identify a predefined image of an object from a number of images of objects in a same category, such as an image of a hammer from a group of images of tools. The user device may access user-specific information and the predefined pass/fail criteria may be based on user-specific information from a user device. For example, the pass/fail criteria for a concentration test may include an ability to identify of an image of a known person from a group of images of people on the user's mobile device (*e.g.,* in a photo album or contact list).

The test may be a math test that includes basic mathematical equations (*e.g*., basic addition, subtraction, multiplication, or division) to test the mental state of the user through testing the mathematical processing of the user. The pass/fail criteria for the math test may include an ability to correctly answer basic mathematical questions, such as "What is 27+36?".

The test may be a test of manual dexterity. The dexterity test may test the mental and/or physical state of the user. The dexterity test may test the physical state of the user by measuring the physical dexterity that may be needed to properly physically handle and interact with diabetes tools and/or objects for treatment. The dexterity test may be tested by a relative change in acceleration or a relative change in orientation of the user device over a period of time while the user holds the device. For example, the user may be asked to perform an action with the mobile device (*e.g*., move up and down, or keep stationary in hand). The pass/fail criteria for the test of manual dexterity may include an ability of the user to trace a displayed shape on the screen of the device and stay within a predefined distance or an ability of the user to touch the location of a sequence of shapes displayed on the screen. The pass/fail criteria for the test of manual dexterity may include a relative change in acceleration or a relative change in orientation of the mobile device over a period of time.

The responses to the baseline test may be received at 208. The responses may be received via the processor of the user device on which the baseline test is provided. The responses may be stored, at 210, on memory of the user device or on remote storage. At 212, the baseline may be established based on the baseline responses. The user response to the baseline test may be stored to establish the baseline for which subsequent tests may be compared. The baseline may be established by comparing the baseline responses to predefined successful responses to the pass/fail criteria. The baseline may be established based on the accuracy of the user. For example, baseline responses may be compared to the predefined responses and the baseline may be set to the accuracy percentage of the user when taking the baseline test. In another example, the baseline may be established based on the successful responses themselves. For example, the baseline responses may be stored, and the baseline may be established as the correct and incorrect responses by the user.

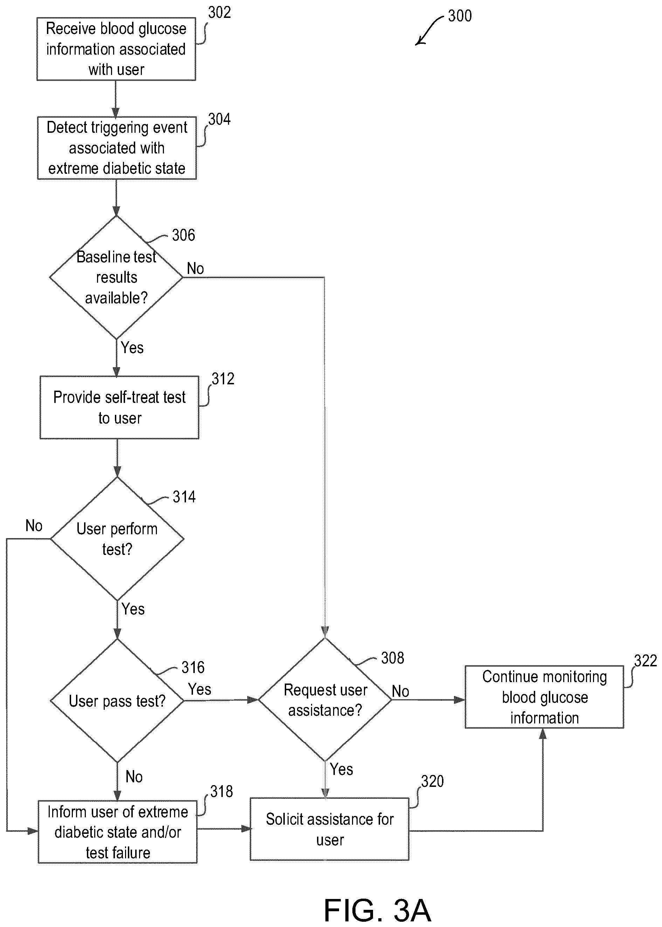

FIG. 3A is a flow diagram of an example method 300 for providing a self-treat test to a user to test the ability of the user to perform self-treatment of a diabetic condition. The method 300 may be performed my one or more computing devices. For example, the method 300 may be performed by a user device, such as the mobile device, the CGM, the BGM, or the insulin pump, or may be distributed across multiple user devices. Though an individual device may be described as performing one or more portions of the method 300, the portions of the method 300 may be performed on another device. For example, though portions of the method 300 may be performed at a user device, the portions may be performed at a remote device and resulting information may be received by the user device on which other portions of the method may be performed.

As shown in FIG. 3A, blood glucose information may be received at 302. The blood glucose information may include the blood glucose levels measured by the BGM or data from the CGM indicating glucose levels of the user. The blood glucose information may be received by a processor of a user device, such as the user's mobile device via a communication circuit. The blood glucose level of the user may be generated from the CGM data by the processor of a CGM device or another user device, such as the mobile device. A triggering event associated with an extreme diabetic state may be detected by the processor of the user device at 304. The triggering event may be detected by the BGM or the CGM and an indication may be communicated to another device, such as the mobile device or another computing device. In another example, the blood glucose level of the user may be transmitted to the mobile device or another device for detecting the triggering event. The triggering event may be detected based on the blood glucose level exceeding a predefined high-end threshold or a predefined low-end threshold. The predefined high-end threshold may indicate that the user has reached a hyperglycemic state. The predefined low-end threshold may indicate that the user has reached a hypoglycemic state. The high-end threshold and/or the low-end threshold may be a predictive threshold that indicates that the user may imminently reach an extreme diabetic state without treatment.

After the triggering event is detected, at 304, an alert may be provided on the user device to inform the user that the user's blood glucose level is above or below the predefined threshold. The alert may be an audible or non-audible alert. The alert may be provided as a notification on a display of the user device that is generated by the processor of the user device. The notification may overlay a lock screen, home screen, or screen of another application executing on the user device. The notification may inform the user that the user is in an extreme diabetic state, or is predicted to be imminently in an extreme diabetic state. The audible alert may be a sound generated by the processor of the user device and transmitted via a local or remote speaker.

After the triggering event is detected, a timer may be started by the processor of the user device. The processor of the user device may await a predefined period of time for input regarding a self-treatment action being taken. This input may be provided from the user confirming that the appropriate self-treatment action has been taken, or the input may be provided in a series of steps, or confirmations, from the user as self-treatment occurs. If positive input fails to be provided within the period of time, a self-treat test may be generated and displayed by the processor to test if the user is mentally fit to perform self-treatment. The predefined period of time for setting the timer for providing the self-treat test may depend on the severity of the glucose level of the user (*e.g.,* a low glucose level of 50 mg/dL may cause the timer to be set to a relatively shorter period of time than a higher glucose level of 79 mg/dL). The timer may be set as a function of the blood-glucose level of the user, after the blood-glucose level of the user reaches a pre-defined high or pre-defined low for the initial triggering event.

At 306, a determination may be made by the processor of the user device as to whether baseline test results are available. The baseline test results may be the results of a baseline test previously performed by the user and stored locally on the user device or at a remote device. In another example, the baseline test results may be predetermined results that establish a baseline against which a user's responses may be compared. If the baseline test results are determined to be available, a self-treat test may be provided by a user device to the user at 312. The self-treat test may include the same, or a subset of, the pass/fail criteria that were included in the baseline test. The self-treat test may be provided to the user via one or more of the user devices.

If it is determined that the user performed the self-treat test at 314 by attempting to complete the one or more pass/fail criteria, a determination may be made by the processor of the user device, at 316, as to whether the user passed the test. The determination may be made by comparing results of the baseline test with the results of the self-treat test. The user may be determined to pass the test if the results of the self-treat test are within a predefined deviation of the baseline test. The predefined deviation may be a percentage or number of correct responses. The predefined deviation may be set to zero, such that the user passes the self-treat test when the successful responses on the self-treat test are the same as the successful responses on the baseline test. The predefined deviation may be a deviation in the user's answers to questions; a deviation in the user's ability to trace a displayed shape on the screen of the user device and stay within a predefined distance; a deviation in the response time for the user selecting the location of a sequence of shapes displayed on the screen; a deviation in the relative change in acceleration or a relative change in orientation of the mobile device while the user is holding the device over a period of time; and/or a deviation in another input received from the user on the user device.

If the user passes the self-treat test, additional tests may be performed to confirm the results of the prior test. For example, the user may perform a dexterity test by being asked via instructions on the user device to hold the user's mobile phone steady for a period of time. The user device's change in orientation or movement may be used to measure the shakiness of the user and infer the severity of the extreme diabetic state and/or the presumed positively-correlated level of mental clarity. Any other test may be performed to confirm the results of the prior test to ensure the user is capable of self-treatment. Though additional tests may be performed, a single test may be performed to expedite the user's ability to treat themselves or request assistance.

If the user fails the test at 316, or the user fails to perform the test at 314, the user may be informed of the extreme diabetic state and/or test failure at 318. For example, the user device may inform the user via a user interface on a display of the user device, audibly by a local speaker on the user device, or via a device external to the user device. The inability to pass the test, or perform the test, may be correlated to a lack of mental ability to self-treat from the extreme diabetic event. Assistance may be solicited for the user by communicating a diabetes-related alert at 320.

The self-treat test may be provided to as a measurement to test the mental and/or physical state of the user. Though pass/fail criteria are described, the test results may indicate a relative level of the user's mental or physical state across a spectrum. The relatively higher the state of confusion (*e.g*., by answering more questions incorrectly), or lower the physical ability of the user (*e.g.,* by being unable to perform physical tests), the less likely or able is the user is to execute a self-treatment event or to execute it effectively (*e.g*., hypo state unawareness, inability to recall strategies, inability to calculate carbs or insulin amounts needed, inability to recall previous actions already executed, inability to recall or execute steps in the right order, etc.). For example, the user may pass four out of five questions to give an indication of the relative mental and/or physical state of the user. The threshold for identifying whether a user passes or fails the self-treat test may be modified (*e.g.,* by the user, a doctor, *etc.*).

If the user fails the self-treat test, additional tests may be performed to confirm the results of the prior test. An additional test may be performed to prevent assistance from being solicited on behalf of the user, when the user is capable of self-treatment. Though additional tests may be performed, a single test may be performed to expedite the user's ability to receive assistance.

The diabetes-related alert may solicit assistance in the treatment of the extreme diabetic state. The diabetes-related alert may be an audible alert generated by the processor of the user device and communicated via a speaker of one or more user devices. The diabetes-related alert may be communicated via one or more remote computing devices external to the user devices. For example, the diabetes-related alert may be communicated via an external speaker device for audible communication to an occupant of a space. The processor of the user device or devices may send a message to the external speaker device via a communication circuit to cause the external speaker device to communicate a predefined message for the diabetes-related alert to occupants of a space. The processor of the user device or devices may send a predefined message via a communication circuit for the diabetes-related alert via a messaging system (*e.g*., instant messenger), email, telecommunication, social media, or text message to a remote device. The processor of the user device or devices may send a message via a communication circuit that is configured to control a home automation system to alert an occupant of a space. For example, the message may be configured to control the lights, blinds, or switching mechanisms for electrical outlets or light switches in the home automation system to alert the user (*e.g.,* using an ambient display). One or more lights (*e.g.,* a red lamp) in a space may be dedicated for alerting occupants of a space regarding the user's extreme diabetic state.

If the user passes the test at 316, a determination may be made at the processor of the user device that the user is capable of self-treating the extreme diabetic state. For example, if the user passes the test it may be assumed that the user has the cognitive ability to self-treat by properly treating the extreme diabetic state to bring the user's blood glucose level back to a normal level. The user may still be asked by the user device whether to solicit assistance. A determination may be made by the processor of the user device, at 308, as to whether the user assistance is requested. For example, a request may be displayed on a user interface of a user device. A determination may be made, based on the user input, to solicit assistance for the user at 320. If the user indicates the user does not want to solicit assistance at 308, or assistance has been solicited at 320, blood glucose information may continue to be monitored at 322 for subsequently providing self-treat tests and/or providing diabetes-related alerts.

If, at 306, the baseline test results are unavailable (*e.g*., the user has not previously performed the baseline test or the predefined baseline test results are unavailable), a determination may be made by the processor of the user device as to whether the user assistance is to be requested at 308. A determination may be made, based on the user input and/or the baseline test results being unavailable, to solicit assistance for the user at 320.

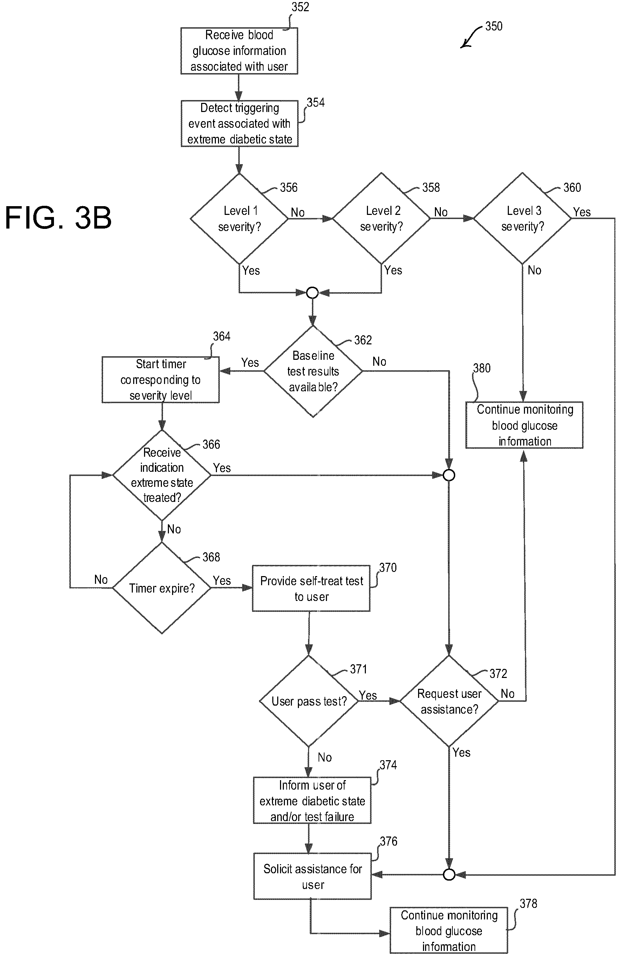

FIG. 3B is a flow diagram of an example method 350 for providing a self-treat test to a user to test the ability of the user to perform self-treatment of a diabetic condition. The method 350 may be performed my one or more computing devices. For example, the method 350 may be performed by a user device, such as the mobile device, the CGM, the BGM, or the insulin pump, or may be distributed across multiple user devices. Though an individual device may be described as performing one or more portions of the method 350, the portions of the method 350 may be performed on another device. For example, though portions of the method 350 may be performed at a user device, the portions may be performed at a remote device and resulting information may be received by the user device on which other portions of the method may be performed.

As shown in FIG. 3B, blood glucose information may be received at 352. The blood glucose information may include the blood glucose levels measured by the BGM or data from the CGM indicating glucose levels of the user. The blood glucose information may be received by a processor of a user device, such as the user's mobile device via a communication circuit. The blood glucose level of the user may be generated from the CGM data by a processor of a CGM device or another user device, such as the mobile device. A triggering event associated with an extreme diabetic state may be detected by the processor of the user device at 354. The triggering event may be detected by the BGM or the CGM and an indication may be communicated to another device, such as the mobile device or another computing device. In another example, the blood glucose level of the user may be transmitted to the mobile device or another device for detecting the triggering event. The triggering event may be detected based on the blood glucose level exceeding a predefined high-end threshold or a predefined low-end threshold. The predefined high-end threshold may indicate that the user has reached a hyperglycemic state. The predefined low-end threshold may indicate that the user has reached a hypoglycemic state. The high-end threshold and/or the low-end threshold may be a predictive threshold that indicates that the user may imminently reach an extreme diabetic state without treatment.

Different thresholds may be defined that correspond to different levels of severity associated with the extreme diabetic state in memory. For example, a first level of severity for a hypoglycemic state may be defined as a measurable glucose concentration of less than 70 mg/dL (3.9 mmol/L). The first level of severity may be defined as a range from an upper limit of 70 mg/dL (3.9 mmol/L) to a lower limit of 54 mg/dL (3.0 mmol/L). A second level of severity for a hypoglycemic state may be defined as a measurable glucose concentration of less than 54 mg/dL (3.0 mmol/L). The second level of severity may be the threshold at which neuroglycopenic symptoms may begin to occur. As such, more immediate action may be taken to resolve the extreme diabetic state. The second level of severity may be a range from an upper limit of 54 mg/dL (3.0 mmol/L) to a lower limit of 42 mg/dL (2.33 mmol/L). In another example, the second level of severity may be a smaller range from an upper limit of 54 mg/dL (3.0 mmol/L) to a lower limit of 53 mg/dL (2.94 mmol/L). The lower limit for the second level of severity may also range anywhere between 53 mg/dL (2.94 mmol/L) and 42 mg/dL (2.33 mmol/L), as an example. In an example in which one of the first or second levels of severity is omitted, a single level of severity may cover a range of the first and second levels of severity described herein. For example, a single level of severity may range from an upper limit of 70 mg/dL (3.9 mmol/L) to a lower limit of 53 mg/dL (2.94 mmol/L), or from an upper limit of 70 mg/dL (3.9 mmol/L) to a lower limit of 42 mg/dL (2.33 mmol/L). Another level of severity, or a third level of severity, for a hypoglycemic state may be defined as a measurable glucose concentration of less than the prior level(s) of severity. The third level of severity may be the threshold at which an extreme diabetic state may alter the mental and/or physical condition of the user. For example, the third level of severity may be defined as less than 53 mg/dL (2.94 mmol/L), less than 42 mg/dL (2.33 mmol/L), or anywhere in between. The third severity level may have a bottom limit, such that it includes a range. For example, the third level of severity may be defined as being between 53 mg/dL (2.94 mmol/L) and 30 mg/dL (1.67 mmol/L), or 42 mg/dL (2.3 mmol/L) and 30 mg/dL (1.67 mmol/L). For the third level of severity, an alert for immediate assistance may be sent. Though example thresholds are provided for different levels of severity for the hypoglycemic state, other thresholds and/or number of levels of severity may be defined.

Different thresholds may similarly be defined for the hyperglycemic state. For example, a first level of severity for a hyperglycemic state may be defined as a measurable glucose concentration of greater than 200 mg/dL (11.1 mmol/L). A second level of severity for a hyperglycemic state may be defined as a measurable glucose concentration of greater than 350 mg/dL (19.4 mmol/L). The second level of severity may be the threshold at which neuroglycopenic symptoms may begin to occur. As such, more immediate action may be taken to resolve the extreme diabetic state. A third level of severity for a hyperglycemic state may be defined as a measurable glucose concentration of greater than 700 mg/dL (38.8 mmol/L). The third level of severity may be the threshold at which an extreme diabetic state may alter the mental and/or physical condition of the user. For the third level of severity, an alert for immediate assistance may be sent. Though example thresholds are provided for different levels of severity for the hyperglycemic state, other thresholds and/or number of levels of severity may be defined.

Though certain thresholds may be used as examples herein, these thresholds may be modified. Different thresholds may be prestored, user-defined, and/or defined by a third party (*e.g.,* a physician) on the user device and stored in memory. For example, the prestored thresholds may be a factory preset stored in the memory of one or more user devices. The prestored thresholds may be modified. The processor of the user device may limit the low-end threshold from being moved below a predefined low-end limit for detecting a hypoglycemic state. The processor of the user device may limit the high-end threshold from being moved above a predefined high-end limit for detecting a hyperglycemic state. The thresholds may be set to the low-end limit and/or the high-end limit as the factory preset stored in memory.

The thresholds may be user-defined to be specific to different users or types of users. Different thresholds may be stored for different users or types of users based on a duration of diabetes, an age/life expectancy, comorbid conditions, known cardiovascular disease or advanced microvascular complications, hypoglycemia unawareness, and/or other individual user considerations. The thresholds may also be determined by a function, which may be different based on different users or types of users.

After a level 1 severity is detected by the processor of the user device, at 356, a determination may be made by the processor at 362 as to whether baseline test results are available in memory. Similarly, after a level 2 severity is detected, at 358, a determination may be made at 362 as to whether the baseline test results are available. The baseline test results for the level 1 severity and may be the same as or different than the baseline test results for the level 2 severity of the extreme diabetic state.

If the baseline test results are determined to be unavailable at 362 (*e.g.,* the user has failed to previously perform the baseline test or the predefined baseline test results are unavailable), a determination may be made at 372 as to whether the user assistance is to be requested. A determination may be made based on the user input and/or the baseline test results being unavailable. For example, the processor of the user device may display a request to the user to ask if the user would like to request assistance. If the user assistance is to be requested, the user assistance may be solicited for the user at 376. The user assistance may be requested by the user device sending a request via a communication circuit to another device.

If the baseline test results are determined to be available in memory, a timer may be started by the processor of the user device at 364. The timer may correspond to the severity level detected for the extreme diabetic state. The timer may be the same or different for the severity level detected for the extreme diabetic state. A longer timer may be triggered after detection of a level 1 severity associated with the extreme diabetic state than for the level 2 severity. For example, a three-minute timer may be triggered after the level 1 severity is detected, while a one-minute timer may be triggered after the level 2 severity is detected. The timer may allow a user time to treat the extreme diabetic state and/or provide information indicating that the treatment has occurred. As different users may react differently to different blood glucose levels, the timer may be user-defined or defined by a third party (*e.g*., such as a physician) on the user device and stored in memory for different levels of severity. The timer may also, or alternatively, be set as a function of the severity level or blood glucose level of the user. For example, a longer timer may be set (*e.g*., established from an upper limit) for lower severity levels, while a shorter timer may be set (*e.g*., established from an upper limit) for higher severity levels.

At 366, a determination may be made by the processor of the user device as to whether an indication has been received that indicates the extreme diabetic state has been treated appropriately. For example, the user may provide information at the user's mobile device, or another computing device, by selecting a button and/or entering text indicating that an appropriate amount of carbohydrates have been consumed or an appropriate amount of glucagon has been administered to treat a hypoglycemic state. The user may also, or alternatively, press a button and/or provide text on the user's mobile device, or another computing device, indicating that an appropriate amount of insulin has been administered to treat a hyperglycemic event. The mobile device or other computing device may receive a message from another user device, such as an insulin pump, CGM, or a BGM, that an appropriate amount of insulin has been a administered or a user's blood-glucose levels have been treated.

Glucose (*e.g.,* 15-20 g) may be a treatment for a conscious individual with a level 1 hypoglycemia. Level 1 hypoglycemia may be detected when the blood glucose level of the user is less than 70 mg/dL (3.9 mmol/L). Any form of carbohydrate that includes glucose may be used for treatment. After a period of time has elapsed (*e.g.,* fifteen minutes) after treatment, if updated blood glucose information shows continued hypoglycemia, the treatment may be repeated. Once the updated blood glucose information indicates that the blood glucose level of the user returns to normal, the user may consume additional glucose to prevent recurrence of hypoglycemia. Glucagon may be prescribed to users at increased risk of level 2 hypoglycemia. Level 2 hypoglycemia may occur when the blood glucose level of the user is less than 54 mg/dL (3.0 mmol/L).

After a user has been treated, hypoglycemia unawareness or one or more episodes of level 3 hypoglycemia may trigger reevaluation of the treatment regimen. Insulin-treated users with hypoglycemia unawareness or an episode of level 2 hypoglycemia may be advised via the display of the user device to raise their glycemic targets to strictly avoid hypoglycemia for at least several weeks in order to partially reverse hypoglycemia unawareness and reduce risk of future episodes.

If an indication is received at 366 indicating that the extreme diabetic state has been treated, a determination may be made by the processor of the user device at 372 as to whether the user assistance is to be requested. A determination may be made based on the user input in response to a request displayed on the user device and/or the indication of the extreme diabetic state being indicated as being treated at 366. If the user assistance is to be requested, the user assistance may be solicited for the user at 376.

The processor of the user device may continue to monitor the expiration of the timer at 368 and if the indication of the extreme diabetic state being treated fails to be received after the expiration of the timer, the processor of the user device may provide a self-treat test to the user at 370. The self-treat test may be displayed on a display of the user device, such as the display of the user's mobile device for example. The self-treat test for a level 1 severity may be the same or different than the self-treat test for a level 2 severity associated with the extreme diabetic state.

At 371, a determination may be made by the processor of the user device as to whether the user passed the self-treat test. At 371, the determination may include determining whether the user attempted one or more pass/fail criteria of the self-treat test. The determination as to whether the user passed the test may be made by comparing results of the baseline test with the results of the self-treat test. The user may be determined to pass the test if the results of the self-treat test are within a predefined deviation of the baseline test. The predefined deviation may be a percentage or number of correct responses. The predefined deviation may be set to zero, such that the user passes the self-treat test when the successful responses on the self-treat test are the same as the successful responses on the baseline test. The predefined deviation may be a deviation in the user's answers to questions; a deviation in the user's ability to trace a displayed shape on the screen of the user device and stay within a predefined distance; a deviation in the response time for the user selecting the location of a sequence of shapes displayed on the screen; a deviation in the relative change in acceleration or a relative change in orientation of the mobile device while the user is holding the device over a period of time; and/or a deviation in another input received from the user on the user device.

If the user passed the self-treat test, additional tests may be performed to confirm the results of the prior test. Though additional tests may be performed, a single test may be performed to expedite the user's ability to treat themselves or request assistance.

The determination at 371 may include determining whether the user passed the self-treat test within a period of time. A test timer may be started by the processor of the user device when the self-treat test is provided to the user. The test timer may be different for different levels of severity and/or blood glucose levels of the user. The test timer may be shorter than the timer at 364, which may be referred to as a self-treat timer. Though the timer at 364 may be set for the amount of time for self-treatment by the user and/or passing of the self-treat test. The test timer may be set to a time limit that is less than the self-treat timer, as the severity of the user's condition may have increased. The test timer may be set as a function of the self-treat timer and/or the severity level. For example, the self-treat timer may be a multiple or an order of magnitude greater that the test timer. The multiple or magnitude may be increased as the severity level of the extreme diabetic state (*e.g*., detected at 356, 358) is decreased. When the extreme diabetic state of the user is detected as being a level 1 severity, the self-treat timer may be set to three minutes and the test timer may be set to twenty seconds, for example. In another example, when the extreme diabetic state of the user is detected as being a level 2 severity, the self-treat timer may be set to one minute and the test timer may be set to ten seconds.

If the user fails the test or fails to perform the test at 371 (*e.g*., within a period of time), the user may be informed of the extreme diabetic state and/or test failure at 374. For example, the processor of the user device may inform the user via a user interface on a display of the user device, audibly by a speaker, or via a device external to the user device. The inability to pass the test, or perform the test, may be correlated to a lack of mental ability to self-treat from the extreme diabetic event. Assistance may be solicited for the user by communicating a diabetes-related alert at 376.

The diabetes-related alert may solicit assistance in the treatment of the extreme diabetic state. The diabetes-related alert may be an audible alert generated by the processor of the user device and communicated via a speaker of one or more user devices. The diabetes-related alert may be communicated via one or more remote computing devices external to the user devices. For example, the diabetes-related alert may be communicated via an external speaker device for audible communication to an occupant of a space. The processor of the user device or devices may send a message to the external speaker device via a communication circuit to cause the external speaker device to communicate a predefined message for the diabetes-related alert to occupants of a space. The processor of the user device or devices may send a predefined message via a communication circuit for the diabetes-related alert via a messaging system (*e.g*., instant messenger), email, telecommunication, social media, or text message to a remote device. The processor of the user device or devices may send a message via a communication circuit that is configured to control a home automation system to alert an occupant of a space. For example, the message may be configured to control the lights, blinds, or switching mechanisms for electrical outlets or light switches in the home automation system to alert the user (*e.g.,* using an ambient display). One or more lights (*e.g.,* a red lamp) in a space may be dedicated for alerting occupants of a space regarding the user's extreme diabetic state.

If the user passes the test at 371, a determination may be made on the processor of the user device that the user is capable of self-treating the extreme diabetic state. For example, if the user passes the test it may be assumed by the user device that the user has the cognitive ability to self-treat by properly treating the extreme diabetic state to bring the user's blood glucose level back to a normal level. The user may still be asked by text on the display or speaker of the user device whether to solicit assistance. A determination may be made by the processor of the user device, at 372, as to whether the user assistance is requested. For example, a request may be displayed by the processor on a user interface of the user device. A determination may be made, based on the user input, to solicit assistance for the user at 372. If the user indicates the user does not want to solicit assistance at 372, or assistance has been solicited at 376, blood glucose information may continue to be monitored at 378, 380 for subsequently providing self-treat tests and/or providing diabetes-related alerts.

Referring back to 354 of the method 300, the detected triggering event associated with the extreme diabetic state may have a higher level of severity. For example, the processor of the user device may detect a level 3 severity at 360. The third level of severity may be the threshold at which an extreme diabetic state may alter the mental and/or physical condition of the user. For the third level of severity, an alert for immediate assistance may be sent. For example, when level 3 severity, or another severity level defined as being similarly extreme, is detected, the processor of the user device may skip any processing or time allocating for enabling self-treatment and/or performance of the self-treat test by the user. The processor of the user device may proceed to solicit assistance of the user at 376. This assistance may be solicited immediately, as the user may be automatically detected as being incapable of self-treatment and/or passing the self-treat test.

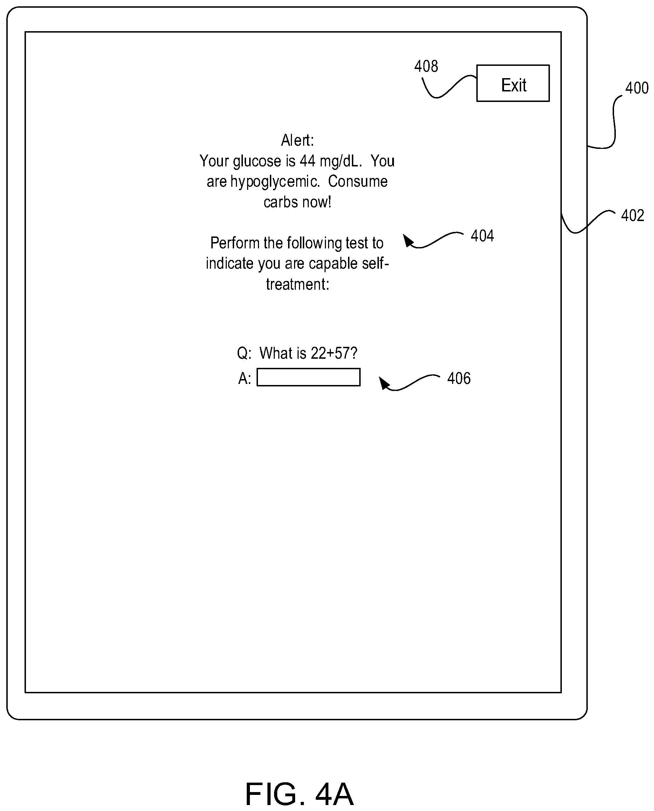

FIGs. 4A-4D depicts example graphical user interfaces (GUIs) that may be displayed on a user device. As shown in FIG. 4A, a GUI 402 may be displayed on a user device 400. The user device 400 may include a mobile device, a CGM, a CGM controller, a BGM, and/or an insulin pump 116, 118. For example, the GUI 402 may be generated by software or firmware on a CGM controller, or in a CGM application on a mobile device. The GUI 402 may be generated locally, or generated on a remote device and displayed via an application, such as a web browser, on the user device 400. The GUI 402 may be overlaid on top of a lock screen, a home screen, or another application being displayed on the user device 400.

The GUI 402 may include an alert 404. The alert 404 may indicate the user's blood glucose level, an indication of a current or predicted extreme diabetic state, and/or an indication to consume carbs or insulin to help self-treat the extreme diabetic state. The alert 404 may request the user to perform a test to indicate the user's ability to perform self-treatment.

The GUI 402 may include a self-treat test 406. The self-treat test may be provided with the alert 404, or after selection (*e.g.,* of a button) by a user to indicate the user would like to take the test. The self-treat test 406 may include one or more pass/fail criteria. The number of pass/fail criteria (*e.g.,* questions, *etc.*) that are displayed to the user on the user device may depend on the type of user device. For example, a tablet or mobile device may have a larger display than a CGM or CGM controller to allow more questions to be displayed to the user. The number of criteria may be automatically determined based on the display size of the user device.

The GUI 402 may include an exit button 408 that may allow the user to exit the test. If the user exits, or does not perform the test over a predefined period of time, a diabetes-related alert may be communicated from the user device 400 to solicit assistance for the user. It may be assumed that the user is incapable of passing the self-treat test 406. The exit button 408 may be inoperable until after the user attempts the self-treat test 406.

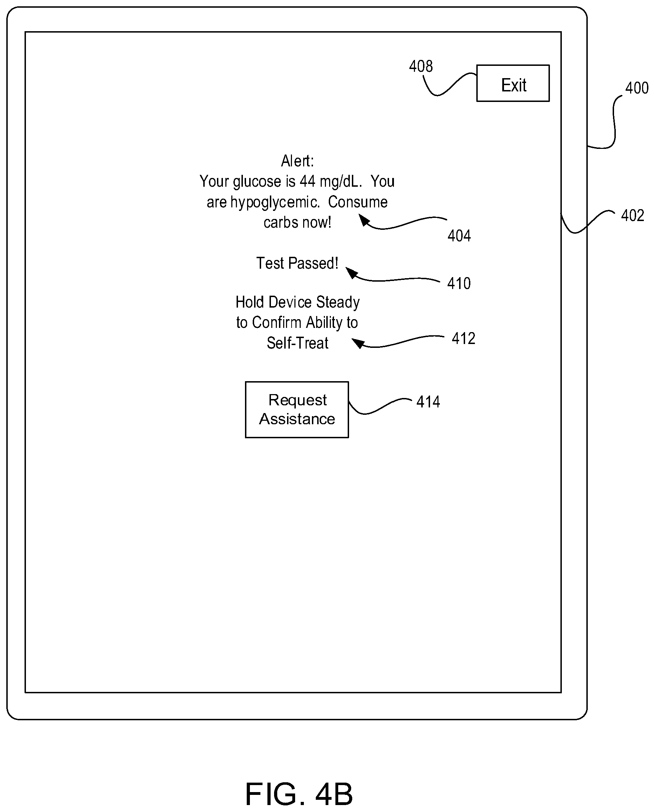

As shown in FIG. 4B, the GUI 402 may include a pass/fail indicator 410 that indicates that the user has passed or failed the self-treat test. If the user has passed the self-treat test, the GUI 402 may request from the user whether the user would like to solicit assistance. For example, the GUI 402 may include a request assistance button 414. Upon receipt of the selection of the request assistance button 414 by the user, the user device 400 may communicate a diabetes-related alert.

The GUI 402 may include an additional test 412 to confirm that the user has the ability to perform self-treatment. For example, the GUI 402 may ask the user to perform a dexterity test by asking the user to hold the user device steady for a period of time. The user device 400 may monitor relative changes in orientation or movement in a direction and determine that the user has passed or failed the test based on whether the orientation or movement is within a predefined threshold for a period of time. The user device's change in orientation or movement may be used to measure the shakiness of the user and infer the severity of the extreme diabetic state and/or the presumed positively-correlated level of mental clarity. Any other test may be performed to confirm the results of the prior test to ensure the user is capable of self-treatment. Though additional tests may be performed, a single test may be performed to expedite the user's ability to self-treat or request assistance.

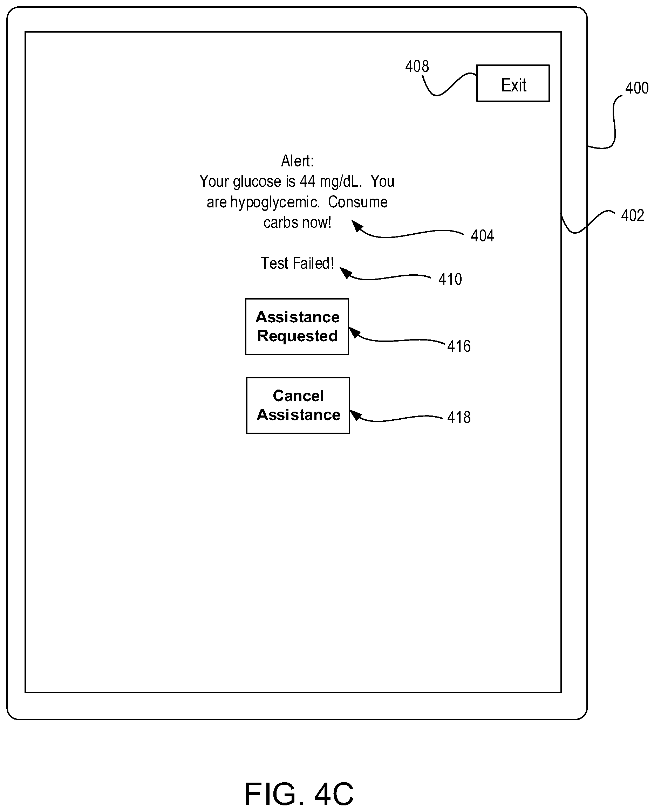

As shown in FIG. 4C, the pass/fail indicator 410 may indicate that the user has failed the self-treat test. If the user has failed the self-treat test, the user device 400 may automatically communicate a diabetes-related alert. The GUI 402 may include an alert indicator 416 that indicates that the diabetes-related alert has been communicated to solicit assistance on behalf of the user. The user may select a cancel assistance button 418 to communicate a cancelation message indicating that the user is capable of self-treatment. Another self-treat test may be displayed to the user to be passed before the cancelation message is sent.

The GUI 402 may include an additional test (not shown) prior to communicating the diabetes related alert to solicit assistance on behalf of the user. An additional test may be performed to prevent assistance from being solicited on behalf of the user, when the user is capable of self-treatment. Though additional tests may be performed, a single test may be performed to expedite the user's ability to receive assistance.

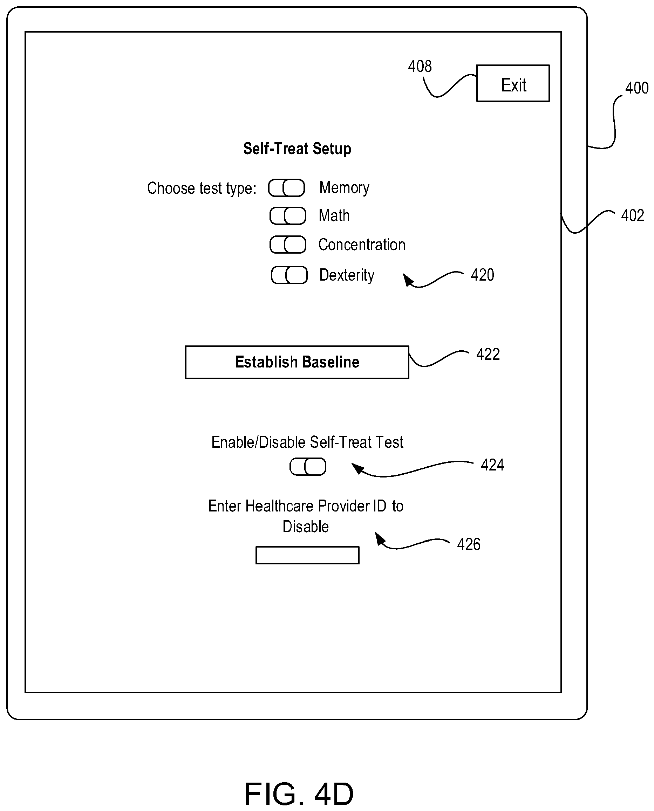

As shown in FIG. 4D, the GUI 402 may include settings for the self-treat test. The settings may be displayed upon an initial execution of an application on the user device 400, or upon selection to view the settings by the user. The settings may include test type options 420. The test type options 420 may allow the user to select the type of test or tests that may be presented. The GUI 402 may include an establish baseline setting 422. Upon selection of the establish baseline setting 422, the user may perform one or more baseline tests of the type(s) 420 that have been selected. The baseline test may also, or alternatively, be performed upon initial execution of the application and the establish baseline setting 422 may be selected to update the baseline test and/or responses.

The GUI 402 may allow the user to enable/disable the self-treat test based on an actuation of the setting 424. The user device may prevent the user from disabling the self-treat test. An identifier and/or password 426 may be entered to disable the self-treat test. The identifier and/or password may be associated with another user, such as a healthcare provider, for example. This may prevent a user that is in an extreme diabetic state from unknowingly disabling the self-treat test and/or emergency alerts associated therewith.

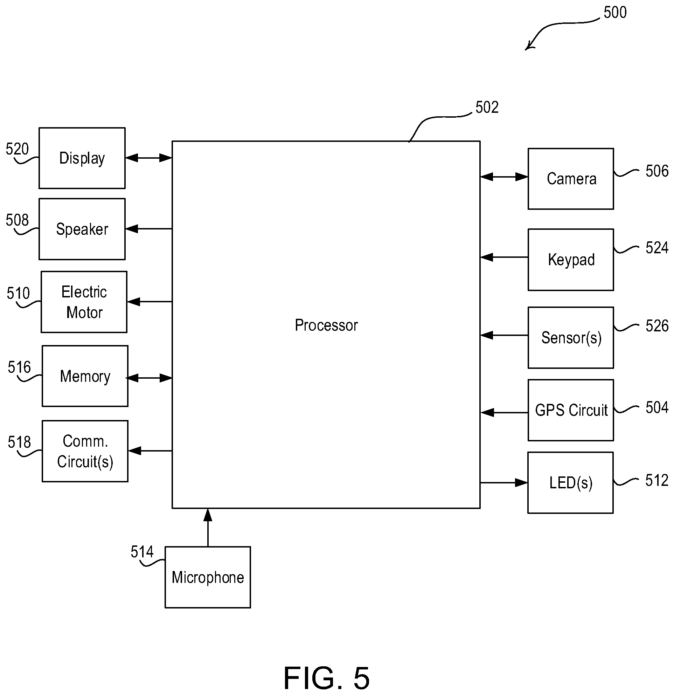

FIG. 5 is a block diagram of an example computing device 500. The computing device may be a mobile computing device, such as a tablet, a cellular phone, a wearable device, a CGM controller device, or another computing device, for example. As shown in FIG. 5, the computing device 500 may include a processor 502 for controlling the functionality of the computing device 500. The processor 502 may include one or more circuits, such as general purpose processors, special purpose processors, conventional processors, digital signal processors (DSPs), microprocessors, integrated circuits, a programmable logic device (PLD), application specific integrated circuits (ASICs), and/or the like. The processor 502 may perform signal coding, data processing, power control, image processing, input/output processing, and/or any other functionality that enables the computing device 500 to perform as described herein.

The processor 502 may store information in and/or retrieve information from the memory 516. The memory 516 may include a non-removable memory and/or a removable memory. The non-removable memory may include random-access memory (RAM), read-only memory (ROM), a hard disk, and/or any other type of non-removable memory storage. The removable memory may include a subscriber identity module (SIM) card, a memory stick, a memory card (*e.g.,* a digital camera memory card), and/or any other type of removable memory. The processor 502 may access the memory 516 for executable instructions and/or other information that may be used by the computing device 500.

The computing device 500 may include a camera 506 that may be in communication with the processor 502. The camera 506 may be a digital camera or other optical device capable of generating images and/or videos (*e.g*., image sequences) for being captured at the computing device 500. The camera 506 may include a lighting device capable of flashing to in response to signals from the processor 502. The lighting device may flash to provide alerts via the camera 506.

The computing device 500 may include one or more communication circuits 518. The processor 502 may be in electrical communication with the communication circuit 518 for sending and/or receiving information. The communication circuit 518 may be capable of performing wired and/or wireless communications. For example, the communication circuit 518 may include one or more radio frequency (RF) transceivers for transmitting and receiving RF signals (*e.g*., BLUETOOTH^{®}, near field communication (NFC), WIFI^{®}, WI-MAX^{®}, cellular, *etc.*) via an antenna, or other communications module capable of performing wireless communications. One or more communication circuits 518 may be capable of performing infrared (IR) communications.

The processor 502 may be in electrical communication with a keypad 524 for providing input to the processor 502. The keypad 524 may include one or more keys for receiving input from a user. The keypad 524 may include hard or soft keys for which the function of the keys may change as a user performs selections.

Other input into the processor 502 may be provided by one or more sensors 526. The sensors 526 may include a motion sensor, a proximity sensor, a heartrate monitoring sensor, an accelerometer, a gyroscope, or another sensor on the computing device. The motion sensor may transmit infrared signals or use image processing to sense movement. The proximity sensor may transmit infrared signals to detect when an object is within a predefined proximity. The heartrate monitoring sensor may implement photoplethysmography to detect the amount of blood flow in the user. The heartrate monitoring sensor may include one or more LED and/or photodiodes to detect the amount of blood flow in the user. The heartrate monitoring sensor may implement infrared technology to detect the amount of blood flow in the user. The heartrate monitoring sensor may take an electrocardiogram (ECG) and detect information about the user's heartrate from the ECG. The accelerometer may measure the non-gravitational acceleration of the computing device 500 in a given direction. The accelerometer may respond to vibrations associated with movement in a given direction. The measurements from the accelerometer may be used by the processor 502 to determine the magnitude and/or direction of the relative movement of the computing device 500, or the user's relative position (*e.g.,* standing, sitting, lying down, *etc.*). The gyroscope may be used to determine the orientation of the computing device 500.

The processor 502 may be in electrical communication with and/or generate images on a display 520 for providing information to a user. The communication between the display 520 and the processor 502 may be a two-way communication, as the display 520 may include a touch screen module capable of receiving information from a user and providing such information to the processor 502. For example, the display 520 may provide soft buttons for selection by a user that are recognized by the touch screen module and provided to the processor 502 as input.

The processor 502 may be in electrical communication with and/or control a speaker 508. The speaker 508 may provide an audible sound (*e.g.,* tone, beep, buzz, *etc.*) in response to a triggering event detected by the processor 502.

The computing device 500 may include an electric motor 510 that may be in electrical communication with and/or controlled by the processor 502. The electric motor 510 may rotate and cause the computing device 500 to vibrate (*e.g.,* to indicate an alert, *etc.*) in response to a triggering event detected by the processor 502. The electric motor 510 may provide an alert to supplement the audible alarm or replace the audible alarm provided by the speaker 508.

The processor 502 may be in electrical communication with and/or receive information from a microphone 514. For example, the processor 502 may receive audio signals via the microphone 514.

The computing device 500 may include a global positioning system (GPS) circuit 504. The GPS circuit 504 may be capable of receiving GPS information. The processor 502 may be capable of determining the GPS coordinates (*e.g*., latitude and longitude) of the computing device 500 based on the GPS information received via the GPS circuit.

The computing device 500 may include a visual indicator, such as one or more one or more light-emitting diodes (LEDs) 512. One or more LEDs 512 may be illuminated or flashed to provide an alert or communicate other information to the user (*e.g.,* low battery, turning on of the device, *etc.*)

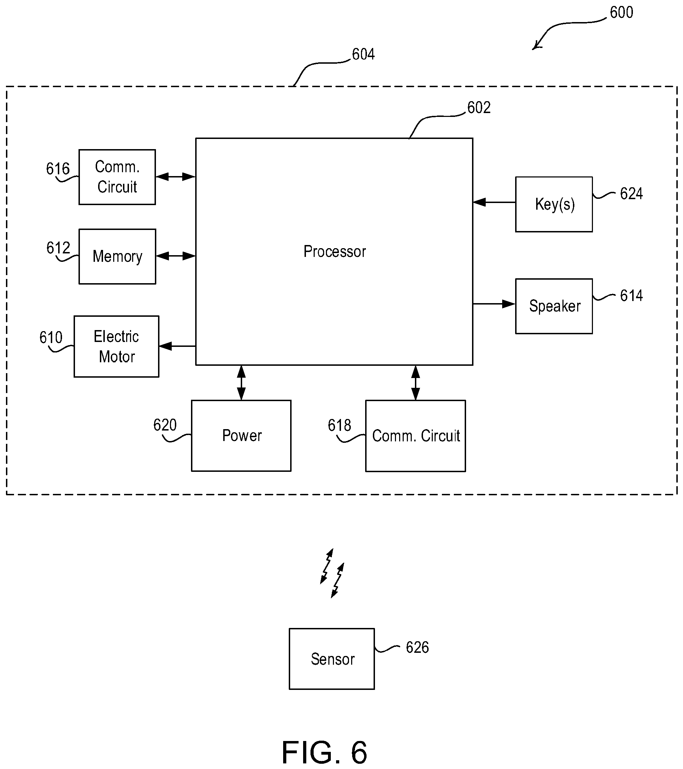

FIG. 6 is a block diagram of an example blood glucose monitoring device 600. The blood glucose monitoring device 600 may be a CGM or FGM, for example. The blood glucose monitoring device 600 may include a subcutaneous sensor 626 that is used to sense and monitor the amount of glucose in interstitial fluid of the user. Data may be transmitted from the sensor 626 to a transmitting device 604. When the blood glucose monitoring device 600 is a CGM, the transmitting device 604 may be located directly over the sensor 626 and may wirelessly power the data transfer from the sensor 626 via power supply 620. When the blood glucose monitoring device 600 is an FGM, the transmitting device 604 may be a mobile device or other reader device that may instantaneously receive the blood glucose information from the sensor 626 when the device is within the RF range of the sensor 626.

The transmitting device 604 may receive data communications from the sensor 626 via a communication circuit 618. The communication circuit 618 may be in electrical communication with a processor 602. The processor 602 may include one or more circuits, such as general purpose processors, special purpose processors, conventional processors, digital signal processors (DSPs), microprocessors, integrated circuits, a programmable logic device (PLD), application specific integrated circuits (ASICs), and/or the like. The processor 602 may perform signal coding, data processing, power control, input/output processing, and/or any other functionality that enables the transmitting device 604 to perform as described herein.

The transmitting device 604 may include another communication circuit 616 for communicating with other devices. The processor 602 may be in electrical communication with the communication circuit 616 for sending and/or receiving information. The communication circuits 616, 618 may be capable of performing wired and/or wireless communications. For example, the communication circuits 616, 618 may include one or more radio frequency (RF) transceivers for transmitting and receiving RF signals (*e.g*., BLUETOOTH^{®}, near field communication (NFC), WIFI^{®}, WI-MAX^{®}, cellular, *etc.*) via an antenna, or other communications module capable of performing wireless communications. The communication circuits 616, 618 may communicate using the same RF protocol or a different RF protocol

The processor 602 may store information in and/or retrieve information from the memory 612. The memory 612 may include a non-removable memory and/or a removable memory. The non-removable memory may include random-access memory (RAM), read-only memory (ROM), a hard disk, and/or any other type of non-removable memory storage. The removable memory may include a subscriber identity module (SIM) card, a memory stick, a memory card (*e.g.,* a digital camera memory card), and/or any other type of removable memory. The processor 602 may access the memory 612 for executable instructions and/or other information that may be used by the transmitting device 604. The processor 602 may be in electrical communication with a one or more input keys 624 for providing input to the processor 602.

The processor 602 may be in electrical communication with and/or control a speaker 614. The speaker 614 may provide an audible sound (*e.g.,* tone, beep, buzz, *etc.*) in response to a triggering event detected by the processor 602.

The blood glucose monitoring device 600 may include an electric motor 610 that may be in electrical communication with and/or controlled by the processor 602. The electric motor 610 may rotate and cause the blood glucose monitoring device 600 to vibrate (*e.g.,* to indicate an alert, *etc.*) in response to a triggering event detected by the processor 602. The electric motor 610 may provide an alert to supplement the audible alarm or replace the audible alarm provided by the speaker 614.

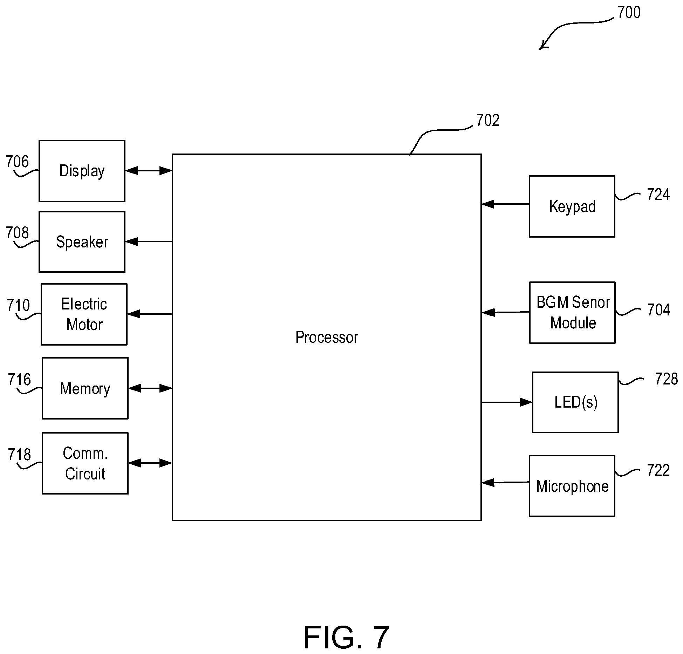

FIG. 7 is a block diagram of an example blood glucose measuring (BGM) device 700. As shown in FIG. 7, the BGM device 700 may include a processor 702 for controlling the functionality of the BGM device 700. The processor 702 may include one or more circuits, such as general purpose processors, special purpose processors, conventional processors, digital signal processors (DSPs), microprocessors, integrated circuits, a programmable logic device (PLD), application specific integrated circuits (ASICs), and/or the like. The processor 702 may perform signal coding, data processing, power control, image processing, input/output processing, and/or any other functionality that enables the BGM device 700 to perform as described herein.

The processor 702 may store information in and/or retrieve information from the memory 716. The memory 716 may include a non-removable memory and/or a removable memory. The non-removable memory may include random-access memory (RAM), read-only memory (ROM), a hard disk, and/or any other type of non-removable memory storage. The removable memory may include a subscriber identity module (SIM) card, a memory stick, a memory card (*e.g.,* a digital camera memory card), and/or any other type of removable memory. The processor 702 may access the memory 716 for executable instructions and/or other information that may be used by the BGM device 700.

The BGM device 700 may include one or more communication circuits 718. The processor 702 may be in electrical communication with the communication circuit 718 for sending and/or receiving information. The communication circuit 718 may be capable of performing wired and/or wireless communications. For example, the communication circuit 718 may include one or more radio frequency (RF) transceivers for transmitting and receiving RF signals (*e.g.,* BLUETOOTH^{®}, near field communication (NFC), WIFI^{®}, WI-MAX^{®}, cellular, *etc.*) via an antenna, or other communications module capable of performing wireless communications. One or more communication circuits 718 may be capable of performing infrared (IR) communications.

The processor 702 may be in electrical communication with a keypad 724 for providing input to the processor 702. The keypad 724 may include one or more keys for receiving input from a user. The keypad 724 may include hard or soft keys for which the function of the keys may change as a user performs selections.

Other input into the processor 702 may be provided by the BGM sensor module 704. The BGM sensor module 704 may include a blood glucose measuring engine that may analyze blood samples provided by a patient on a blood glucose measurement strip and measures the amount of blood glucose in the samples.

The processor 702 may be in electrical communication with and/or generate images on a display 706 for providing information to a user. The communication between the display 706 and the processor 702 may be a two-way communication, as the display 706 may include a touch screen module capable of receiving information from a user and providing such information to the processor 702. For example, the display 706 may provide soft buttons for selection by a user that are recognized by the touch screen module and provided to the processor 702 as input.

The processor 702 may be in electrical communication with and/or control a speaker 708. The speaker 708 may provide an audible sound (*e.g.,* tone, beep, buzz, *etc.*) in response to a triggering event detected by the processor 702.

The BGM device 700 include an electric motor 710 that may be in electrical communication with and/or controlled by the processor 702. The electric motor 710 may rotate and cause the BGM device 700 to vibrate (*e.g.,* to indicate an alert, *etc.*) in response to a triggering event detected by the processor 702. The electric motor 710 may provide an alert to supplement the audible alarm or replace the audible alarm provided by the speaker 708.

The processor 702 may be in electrical communication with and/or receive information from a microphone 722. For example, the processor 702 may receive audio signals via the microphone 722.

The BGM device 700 may include a visual indicator, such as one or more one or more light-emitting diodes (LEDs) 728. One or more LEDs 728 may be illuminated or flashed to provide an alert or communicate other information to the user (*e.g.,* low battery, turning on of the device, *etc.*).

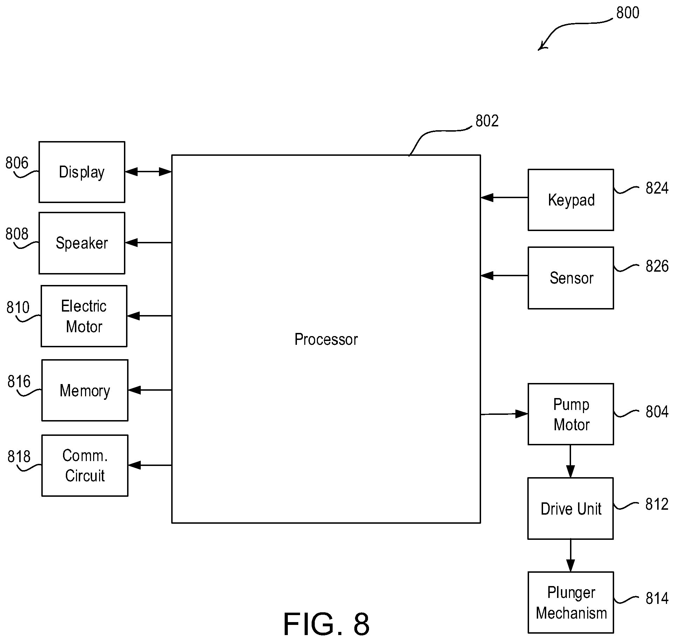

FIG. 8 is a block diagram illustrating an example of an insulin pump 800. As shown in FIG. 8, the insulin pump 800 may include a processor 802. The processor 802 may include one or more circuits, such as general purpose processors, special purpose processors, conventional processors, digital signal processors (DSPs), microprocessors, integrated circuits, a programmable logic device (PLD), application specific integrated circuits (ASICs), and/or the like. The processor 802 may perform signal coding, data processing, power control, image processing, input/output processing, and/or any other functionality that enables the insulin pump 800 to perform as described herein.

The processor 802 may be in electrical communication with and/or control a pump motor 804 in the insulin pump 800. The pump motor 804 may drive a drive unit 812 that pushes a plunger mechanism 814. The plunger mechanism 814 may eject insulin from an insulin cartridge (not shown). The insulin cartridge may include a supply of insulin for delivery to a user.

The processor 802 may be in electrical communication with and/or generate images on a display 806 for providing information to a user. The communication between the display 806 and the processor 802 may be a two-way communication, as the display 806 may include a touch screen module capable of receiving information from a user and providing such information to the processor 802. For example, the display 806 may provide soft buttons for selection by a user that are recognized by the touch screen module and provided to the processor 802 as input.

The processor 802 may be in electrical communication with and/or control a speaker 808. The speaker 808 may provide an audible sound (*e.g.,* tone, beep, buzz, *etc.*) in response to a triggering event detected by the processor 802.

The insulin pump 800 may include an electric motor 810 that may be in electrical communication with and/or controlled by the processor 802. The electric motor 810 may rotate and cause the insulin pump to vibrate (*e.g.,* to indicate an alert, *etc.*) in response to a triggering event detected by the processor 802. The electric motor 810 may provide an alert to supplement the audible alarm or replace the audible alarm provided by the speaker 808.

The processor 802 may be in electrical communication with a memory 816. The processor may store information in and/or retrieve information from the memory 816. The memory 816 may include a non-removable memory and/or a removable memory for storing computer-readable media. The non-removable memory may include random-access memory (RAM), read-only memory (ROM), a hard disk, and/or any other type of non-removable memory storage. The removable memory may include a subscriber identity module (SIM) card, a memory stick, a memory card (*e.g.,* a digital camera memory card), and/or any other type of removable memory. The processor 802 may access the memory 816 for executable instructions and/or other information that may be used by the insulin pump 800.

The insulin pump 800 may include a communication circuit 818. The processor 802 may be in electrical communication with the communication circuit 818 for sending and/or receiving information. The communication circuit 818 may be capable of performing wired and/or wireless communications. For example, the wireless communications circuit 818 may include a radio frequency (RF) transceiver for transmitting and receiving RF signals (*e.g*., BLUETOOTH^{®}), near field communication (NFC), WIFI^{®}, WI-MAX^{®}, cellular, *etc.*) via an antenna, or other communications module capable of performing wireless communications. The communication circuit 818 may be capable of performing infrared (IR) communications.

The processor 802 may be in electrical communication with a keypad 824 for providing input to the processor 802. The keypad 824 may include one or more keys for receiving input from a user. The keypad 824 may include hard or soft keys for which the function of the keys may change as a user performs selections.

Other input into the processor 802 be provided by sensors 826. The sensor 826 may include a pressure sensor that is sensitive to the pressure within a reservoir of insulin; a cartridge sensor that is sensitive to the presence of an insulin cartridge and/or a motion sensor that detects the motion of a gear (not shown) in the drive unit 812.

## Claims

1. A device configured to:
detect (202), via first information from a glucose monitoring device, that a blood-glucose level of a user is between a high-end threshold and a low-end threshold;
after the blood-glucose level of the user is detected between the high-end threshold and the low-end threshold, provide (206) on a mobile device (104) a baseline test, wherein the baseline test comprises at least one predefined pass/fail criteria;
establish a baseline (212) associated with the user based on a response to the baseline test on the mobile device;
detect (304), via second information from the blood glucose monitoring device, an extreme diabetic state in response to the blood-glucose level of the user being above the high-end threshold or below the low-end threshold;
in response to the detecting of the extreme diabetic state, provide (312) on the mobile device a self-treat test, wherein the self-treat test comprises the same or a subset of the at least one predefined pass/fail criteria;
communicate (320) a diabetes-related alert configured to solicit assistance in treatment of the extreme diabetic state:
i) in response to detecting (316) a failure of the self-treat test, wherein the failure of the self-treat test is determined by a predefined deviation between the response to the baseline test and the response to the self-treat test; and
ii) in the absence (314) of a user response to the self-treat test within a predefined period of time corresponding to a level of severity and/or blood-glucose level of the user.

2. The device of claim 1, wherein the self-treat test and the baseline test are the same.

3. The device of claim 1 or claim 2, further configured to:
in response to the self-treat test being passed, determine that the user is capable of self-treating the extreme diabetic state and continue to monitor (322) the blood-glucose level of the user for subsequently providing the self-treat test or communicate the diabetes-related alert.

4. The device of any one of the preceding claims,
wherein the baseline test and the self-treat test each comprise a cognitive test to detect a cognitive ability of the user, wherein the at least one pass/fail criteria comprise at least one question, and wherein the response to the baseline test and the response to the self-treat test each comprise an indication of at least one answer to the at least one question;
wherein, optionally, the device is further configured to provide the baseline test and/or the self-treat test by overlaying the at least one question on a lock screen, a home screen, or another application displayed on the mobile device;
wherein, optionally, the at least one pass/fail criteria comprise a relative change in acceleration or a relative change in orientation of the mobile device over a period of time;
wherein, optionally, the response to the baseline test and the response to the self-treat test each comprise an indication of the acceleration of the mobile device based on accelerometer data or an indication of the orientation of the mobile device based on gyroscope data;
wherein, optionally, the at least one predefined pass/fail criteria comprise a traceable object or a touchable object on a display of the mobile device, and wherein the response to the baseline test and the response to the self-treat test each comprise an indication of at least one user input relative to the traceable object or the touchable object on the display of the mobile device.

5. The device of any one of the preceding claims,
wherein the extreme diabetic state comprises a hypoglycemic event or a hyperglycemic event; and/or
wherein the extreme diabetic state is detected at the high-end threshold or the low-end threshold prior to a threshold for a hypoglycemic event or a hyperglycemic event; and/or
wherein the diabetes-related alert is an audible alert communicated via a speaker of the mobile device.

6. The device of any one of the preceding claims, further configured to:
display a request (308) to communicate the diabetes-related alert on the mobile device in response to the self-treat test being passed; and
communicate the diabetes-related alert in response to receipt of a user indication to communicate the diabetes-related alert.

7. The device of any one of the preceding claims, further configured to:
start a timer (364) at the mobile device, and wherein the self-treat test is provided after an expiration of the timer;
wherein, optionally, the timer corresponds to a severity level of the extreme diabetic state.

8. The device of any one of the preceding claims, further configured to:
start a first timer at the mobile device;
fail to receive an indication that the user has treated the extreme diabetic state prior to an expiration of the first timer; and
start a second timer at the mobile device, wherein the self-treat test is provided after an expiration of the second timer.

9. A device configured to:
detect (202), via first information from a blood glucose monitoring device, that a blood-glucose level of a user is between a high-end threshold and a low-end threshold;
after the blood-glucose level of the user is detected between the high-end threshold and the low-end threshold, provide (206) on a mobile device (104) a baseline test comprising at least one predefined pass/fail criteria;
establish a baseline (212) associated with the user based on the response to the baseline test on the mobile device;
detect (304), via second information from the blood glucose monitoring device, an extreme diabetic state in response to the blood-glucose level of the user being above the high-end threshold or below the low-end threshold;
in response to the detecting of the extreme diabetic state, provide (312) on the mobile device a self-treat test, wherein the self-treat test comprises the same or a subset of the at least one predefined pass/fail criteria ;
provide an additional test to confirm findings of the first test in response to a failure of the self-treat test, wherein the failure of the self-treat test is determined by a predefined deviation between the response to the baseline test and the response to the self-treat test;
communicate (320) a diabetes-related alert configured to solicit assistance in treatment of the extreme diabetic state:
i) in response to detecting (316) the failure of the self-treat test and a failure of the additional test; and
ii) in the absence (314) of a user response to the self-treat test within a predefined period of time corresponding to a level of severity and/or blood-glucose level of the user.

10. The device of claim 9, wherein the self-treat test and the baseline test are the same.

11. The device of claim 9 or claim 10,
wherein the baseline test and the self-treat test each comprise a cognitive test to detect a cognitive ability of the user, wherein the at least one pass/fail criteria comprise at least one question, and wherein the response to the baseline test and the response to the self-treat test each comprise an indication of at least one answer to the at least one question;
wherein, optionally, the device is further configured to provide the baseline test and/or the self-treat test by overlaying the at least one question on a lock screen, a home screen, or another application displayed on the mobile device;
wherein, optionally, the device is further configured to provide the additional test by monitoring a relative change in acceleration of the mobile device associated with the user over a period of time or a relative change in orientation of the mobile device over a period of time, and wherein the failure of the additional test is detected in response to the relative change in acceleration or the relative change in orientation exceeding a baseline threshold.

12. The device of any one of claims 9 to 11,
wherein the device is further configured to provide the additional test by displaying a traceable object or a touchable object on the display of the mobile device, and wherein the failure of the additional test is detected based on an indication of at least one user input relative to the traceable object or the touchable object on the display of the mobile device; and/or
wherein the extreme diabetic state comprises a hypoglycemic event or a hyperglycemic event; and/or
wherein the extreme diabetic state is detected at the high-end threshold or the low-end threshold prior to a threshold for a hypoglycemic event or a hyperglycemic event; and/or
wherein the diabetes-related alert is an audible alert communicated via a speaker of the mobile device.

13. The device of any one of claims 9 to 12, further configured to:
display a request (308) to communicate the diabetes-related alert on the mobile device in response to the self-treat test or the additional test being passed; and
communicate the diabetes-related alert in response to receipt of a user indication to communicate the diabetes-related alert.

14. The device of any one of the preceding claims, wherein the device is further configured to communicate the diabetes-related alert by at least one of the following:
sending a first predefined message for the diabetes-related alert to an external speaker device for audible communication to an occupant of a space;
sending a second predefined message for the diabetes-related alert via telecommunication or text message to an alternate device to the mobile device; or
sending an alert message to control a home automation system to alert the user.

15. The device of claim 9,
wherein the baseline test comprises at least one predefined pass/fail criteria comprises a cognitive test comprising at least one question;
wherein the baseline indicates a cognitive ability of the user;
wherein the response to the baseline test comprises a response to the at least one question of the cognitive test;
wherein the additional test comprises a dexterity test provided by displaying at least one of a traceable object or a touchable object on the mobile device and detecting an indication of at least one user input relative to the traceable object or the touchable object on the display of the mobile device.

## Patentansprüche

1. Vorrichtung, die zu Folgendem konfiguriert ist:
Erkennen (202), über erste Informationen von einer Glukoseüberwachungsvorrichtung, dass ein Blutglukosespiegel eines Benutzers zwischen einem oberen Schwellenwert und einem unteren Schwellenwert liegt;
Bereitstellen (206) eines Basislinientests auf einer mobilen Vorrichtung (104), nachdem der Blutglukosespiegel des Benutzers zwischen dem oberen Schwellenwert und dem unteren Schwellenwert erkannt wurde, wobei der Basislinientest mindestens ein vordefiniertes Bestehen-/Nichtbestehen-Kriterium umfasst;
Erstellen einer Basislinie (212), die dem Benutzer zugeordnet ist, basierend auf einer Reaktion auf den Basislinientest auf der mobilen Vorrichtung;
Erkennen (304), über zweite Informationen von der Blutglukoseüberwachungsvorrichtung, eines extremen diabetischen Zustands als Reaktion darauf, dass der Blutglukosespiegel des Benutzers über dem oberen Schwellenwert oder unter dem unteren Schwellenwert liegt;
Bereitstellen (312) eines Selbstbehandlungstests auf der mobilen Vorrichtung als Reaktion auf das Erkennen des extremen diabetischen Zustands, wobei der Selbstbehandlungstest dasselbe mindestens eine vordefinierte Bestehen-/Nichtbestehen-Kriterium oder eine Teilmenge davon umfasst;
Übermitteln (320) einer diabetesbezogenen Warnung, die dazu konfiguriert ist, Hilfe bei der Behandlung des extremen diabetischen Zustands zu erbitten:
i) als Reaktion auf das Erkennen (316) eines Nichtbestehens des Selbstbehandlungstests, wobei das Nichtbestehen des Selbstbehandlungstests durch eine vordefinierte Abweichung zwischen der Reaktion auf den Basislinientest und der Reaktion auf den Selbstbehandlungstest bestimmt wird; und
ii) in Abwesenheit (314) einer Benutzerreaktion auf den Selbstbehandlungstest innerhalb eines vordefinierten Zeitraums, der einem Schweregrad und/oder einem Blutglukosespiegel des Benutzers entspricht.

2. Vorrichtung nach Anspruch 1, wobei der Selbstbehandlungstest und der Basislinientest derselbe sind.

3. Vorrichtung nach Anspruch 1 oder Anspruch 2, die ferner zu Folgendem konfiguriert ist:
als Reaktion darauf, dass der Selbstbehandlungstest bestanden ist, Bestimmen, dass der Benutzer in der Lage ist, den extremen diabetischen Zustand selbst zu behandeln, und Fortsetzen des Überwachens (322) des Blutglukosespiegels des Benutzers zum anschließenden Bereitstellen des Selbstbehandlungstests oder Übermitteln der diabetesbezogenen Warnung.

4. Vorrichtung nach einem der vorhergehenden Ansprüche,
wobei der Basislinientest und der Selbstbehandlungstest jeweils einen kognitiven Test zum Erkennen einer kognitiven Fähigkeit des Benutzers umfassen, wobei das mindestens eine Bestehen-/Nichtbestehen-Kriterium mindestens eine Frage umfasst und wobei die Reaktion auf den Basislinientest und die Reaktion auf den Selbstbehandlungstest jeweils eine Angabe mindestens einer Antwort auf die mindestens eine Frage umfassen;
wobei die Vorrichtung optional ferner dazu konfiguriert ist, den Basislinientest und/oder den Selbstbehandlungstest durch Überlagern der mindestens einen Frage auf einem Sperrbildschirm, einem Startbildschirm oder einer anderen Anwendung, die auf der mobilen Vorrichtung angezeigt wird, bereitzustellen;
wobei das mindestens eine Bestehen-/Nichtbestehen-Kriterium optional eine relative Änderung der Beschleunigung oder eine relative Änderung der Ausrichtung der mobilen Vorrichtung über einen Zeitraum umfasst;
wobei die Reaktion auf den Basislinientest und die Reaktion auf den Selbstbehandlungstest optional jeweils eine Angabe der Beschleunigung der mobilen Vorrichtung basierend auf Beschleunigungsmesserdaten oder eine Angabe der Ausrichtung der mobilen Vorrichtung basierend auf Gyroskopdaten umfassen;
wobei das mindestens eine vordefinierte Bestehen-/Nichtbestehen-Kriterium optional ein verfolgbares Objekt oder ein berührbares Objekt auf einer Anzeige der mobilen Vorrichtung umfasst und wobei die Reaktion auf den Basislinientest und die Reaktion auf den Selbstbehandlungstest jeweils eine Angabe mindestens einer Benutzereingabe in Bezug auf das verfolgbare Objekt oder das berührbare Objekt auf der Anzeige der mobilen Vorrichtung umfassen.

5. Vorrichtung nach einem der vorhergehenden Ansprüche,
wobei der extreme diabetische Zustand ein hypoglykämisches Ereignis oder ein hyperglykämisches Ereignis umfasst; und/oder
wobei der extreme diabetische Zustand bei dem oberen Schwellenwert oder dem unteren Schwellenwert vor einem Schwellenwert für ein hypoglykämisches Ereignis oder ein hyperglykämisches Ereignis erkannt wird; und/oder
wobei die diabetesbezogene Warnung eine hörbare Warnung ist, die über einen Lautsprecher der mobilen Vorrichtung übermittelt wird.

6. Vorrichtung nach einem der vorhergehenden Ansprüche, die ferner zu Folgendem konfiguriert ist:
Anzeigen einer Aufforderung (308) zum Übermitteln der diabetesbezogenen Warnung auf der mobilen Vorrichtung als Reaktion darauf, dass der Selbstbehandlungstest bestanden ist; und
Übermitteln der diabetesbezogenen Warnung als Reaktion auf den Empfang einer Benutzerangabe zum Übermitteln der diabetesbezogenen Warnung.

7. Vorrichtung nach einem der vorhergehenden Ansprüche, die ferner zu Folgendem konfiguriert ist:
Starten eines Zeitgebers (364) an der mobilen Vorrichtung, und wobei der Selbstbehandlungstest nach einem Ablauf des Zeitgebers bereitgestellt wird;
wobei der Zeitgeber optional einem Schweregrad des extremen diabetischen Zustands entspricht.

8. Vorrichtung nach einem der vorhergehenden Ansprüche, die ferner zu Folgendem konfiguriert ist:
Starten eines ersten Zeitgebers an der mobilen Vorrichtung;
Nichtempfangen einer Angabe, dass der Benutzer den extremen diabetischen Zustand behandelt hat, vor einem Ablauf des ersten Zeitgebers; und
Starten eines zweiten Zeitgebers an der mobilen Vorrichtung, wobei der Selbstbehandlungstest nach einem Ablauf des zweiten Zeitgebers bereitgestellt wird.

9. Vorrichtung, die zu Folgendem konfiguriert ist:
Erkennen (202), über erste Informationen von einer Blutglukoseüberwachungsvorrichtung, dass ein Blutglukosespiegel eines Benutzers zwischen einem oberen Schwellenwert und einem unteren Schwellenwert liegt;
Bereitstellen (206) eines Basislinientests auf einer mobilen Vorrichtung (104), der mindestens ein vordefiniertes Bestehen-/Nichtbestehen-Kriterium umfasst, nachdem der Blutglukosespiegel des Benutzers zwischen dem oberen Schwellenwert und dem unteren Schwellenwert erkannt wurde;
Erstellen einer Basislinie (212), die dem Benutzer zugeordnet ist, basierend auf der Reaktion auf den Basislinientest auf der mobilen Vorrichtung;
Erkennen (304) eines extremen diabetischen Zustands anhand von zweiten Informationen von der Blutglukoseüberwachungsvorrichtung als Reaktion darauf, dass der Blutglukosespiegel des Benutzers über dem oberen Schwellenwert oder unter dem unteren Schwellenwert liegt;
Bereitstellen (312) eines Selbstbehandlungstests auf der mobilen Vorrichtung als Reaktion auf das Erkennen des extremen diabetischen Zustands, wobei der Selbstbehandlungstest dasselbe mindestens eine vordefinierte Bestehen-/Nichtbestehen-Kriterium oder eine Teilmenge davon umfasst;
Bereitstellen eines zusätzlichen Tests zum Bestätigen der Ergebnisse des ersten Tests als Reaktion auf ein Nichtbestehen des Selbstbehandlungstests, wobei das Nichtbestehen des Selbstbehandlungstests durch eine vordefinierte Abweichung zwischen der Reaktion auf den Basislinientest und der Reaktion auf den Selbstbehandlungstest bestimmt wird;
Übermitteln (320) einer diabetesbezogenen Warnung, die dazu konfiguriert ist, Hilfe bei der Behandlung des extremen diabetischen Zustands zu erbitten:
i) als Reaktion auf das Erkennen (316) des Nichtbestehens des Selbstbehandlungstests und eines Nichtbestehens des zusätzlichen Tests; und
ii) in Abwesenheit (314) einer Benutzerreaktion auf den Selbstbehandlungstest innerhalb eines vordefinierten Zeitraums, der einem Schweregrad und/oder einem Blutglukosespiegel des Benutzers entspricht.

10. Vorrichtung nach Anspruch 9, wobei der Selbstbehandlungstest und der Basislinientest derselbe sind.

11. Vorrichtung nach Anspruch 9 oder Anspruch 10,
wobei der Basislinientest und der Selbstbehandlungstest jeweils einen kognitiven Test zum Erkennen einer kognitiven Fähigkeit des Benutzers umfassen, wobei das mindestens eine Bestehen-/Nichtbestehen-Kriterium mindestens eine Frage umfasst und wobei die Reaktion auf den Basislinientest und die Reaktion auf den Selbstbehandlungstest jeweils eine Angabe mindestens einer Antwort auf die mindestens eine Frage umfassen;
wobei die Vorrichtung optional ferner dazu konfiguriert ist, den Basislinientest und/oder den Selbstbehandlungstest durch Überlagern der mindestens einen Frage auf einem Sperrbildschirm, einem Startbildschirm oder einer anderen Anwendung, die auf der mobilen Vorrichtung angezeigt wird, bereitzustellen;
wobei die Vorrichtung optional ferner dazu konfiguriert ist, den zusätzlichen Test durch Überwachen einer relativen Änderung der Beschleunigung der mobilen Vorrichtung, die dem Benutzer zugeordnet ist, über einen Zeitraum oder einer relativen Änderung der Ausrichtung der mobilen Vorrichtung über einen Zeitraum bereitzustellen, und wobei das Nichtbestehen des zusätzlichen Tests als Reaktion darauf erkannt wird, dass die relative Änderung der Beschleunigung oder die relative Änderung der Ausrichtung einen Basislinienschwellenwert überschreitet.

12. Vorrichtung nach einem der Ansprüche 9 bis 11,
wobei die Vorrichtung ferner dazu konfiguriert ist, den zusätzlichen Test durch Anzeigen eines verfolgbaren Objekts oder eines berührbaren Objekts auf der Anzeige der mobilen Vorrichtung bereitzustellen, und wobei das Nichtbestehen des zusätzlichen Tests auf der Grundlage einer Anzeige mindestens einer Benutzereingabe in Bezug auf das verfolgbare Objekt oder das berührbare Objekt auf der Anzeige der mobilen Vorrichtung erkannt wird; und/oder
wobei der extreme diabetische Zustand ein hypoglykämisches Ereignis oder ein hyperglykämisches Ereignis umfasst; und/oder
wobei der extreme diabetische Zustand bei dem oberen Schwellenwert oder dem unteren Schwellenwert vor einem Schwellenwert für ein hypoglykämisches Ereignis oder ein hyperglykämisches Ereignis erkannt wird; und/oder
wobei die diabetesbezogene Warnung eine akustische Warnung ist, die über einen Lautsprecher der mobilen Vorrichtung übermittelt wird.

13. Vorrichtung nach einem der Ansprüche 9 bis 12, die ferner zu Folgendem konfiguriert ist:
Anzeigen einer Aufforderung (308) zum Übermitteln der diabetesbezogenen Warnung auf der mobilen Vorrichtung als Reaktion darauf, dass der Selbstbehandlungstest oder der zusätzliche Test bestanden ist; und
Übermitteln der diabetesbezogenen Warnung als Reaktion auf den Empfang einer Benutzerangabe zum Übermitteln der diabetesbezogenen Warnung.

14. Vorrichtung nach einem der vorhergehenden Ansprüche, wobei die Vorrichtung ferner zum Übermitteln der diabetesbezogenen Warnung durch mindestens eines der Folgenden konfiguriert ist:
Senden einer ersten vordefinierten Nachricht für die diabetesbezogene Warnung an eine externe Lautsprechervorrichtung zur hörbaren Übermittlung an einen in einem Raum Anwesenden;
Senden einer zweiten vordefinierten Nachricht für die diabetesbezogene Warnung über Telekommunikation oder Textnachricht an eine alternative Vorrichtung zur mobilen Vorrichtung; oder
Senden einer Warnnachricht zum Steuern eines Heimautomatisierungssystems, um den Benutzer zu warnen.

15. Vorrichtung nach Anspruch 9,
wobei der Basislinientest mindestens ein vordefiniertes Bestehen-/Nichtbestehen-Kriterium umfasst, das einen kognitiven Test umfasst, der mindestens eine Frage umfasst;
wobei die Basislinie eine kognitive Fähigkeit des Benutzers angibt;
wobei die Reaktion auf den Basislinientest eine Reaktion auf die mindestens eine Frage des kognitiven Tests umfasst;
wobei der zusätzliche Test einen Geschicklichkeitstest umfasst, der durch Anzeigen mindestens eines von einem verfolgbaren Objekt oder einem berührbaren Objekt auf der mobilen Vorrichtung und Erkennen einer Angabe mindestens einer Benutzereingabe in Bezug auf das verfolgbare Objekt oder das berührbare Objekt auf der Anzeige der mobilen Vorrichtung bereitgestellt wird.

## Revendications

1. Dispositif configuré pour :
détecter (202), par l'intermédiaire d'une première information provenant d'un dispositif de surveillance de glucose, qu'un taux de glucose sanguin d'un utilisateur est entre un seuil maximal et un seuil minimal ;
après la détection du taux de glucose sanguin de l'utilisateur entre le seuil maximal et le seuil minimal, fournir (206) sur un dispositif mobile (104) un test de référence, dans lequel le test de référence comprend au moins un critère de réussite/échec prédéfini ;
établir une référence (212) associée à l'utilisateur en se basant sur une réponse au test de référence sur le dispositif mobile ;
détecter (304), par l'intermédiaire d'une seconde information provenant du dispositif de surveillance de glucose sanguin, un état diabétique extrême en réponse au fait que le taux de glucose sanguin de l'utilisateur est supérieur au seuil maximal ou inférieur au seuil minimal ;
en réponse à la détection de l'état diabétique extrême, fournir (312) sur le dispositif mobile un test d'auto-soin, dans lequel le test d'auto-soin comprend le même ou un sous-ensemble de l'au moins un critère de réussite/échec prédéfini ;
communiquer (320) une alerte liée au diabète configurée pour solliciter une assistance dans le traitement de l'état diabétique extrême :
i) en réponse à la détection (316) d'un échec du test d'auto-soin, dans lequel l'échec du test d'auto-soin est déterminé par un écart prédéfini entre la réponse au test de référence et la réponse au test d'auto-soin ; et
ii) en l'absence (314) d'une réponse de l'utilisateur au test d'auto-soin dans une période de temps prédéfinie correspondant à un degré de sévérité et/ou au taux de glucose sanguin de l'utilisateur.

2. Dispositif selon la revendication 1, dans lequel le test d'auto-soin et le test de référence sont identiques.

3. Dispositif selon la revendication 1 ou la revendication 2, en outre configuré pour :
en réponse à la réussite du test d'auto-soin, déterminer que l'utilisateur est capable d'auto-soigner l'état diabétique extrême et continuer à surveiller (322) le taux de glucose sanguin de l'utilisateur pour fournir ensuite le test d'auto-soin ou communiquer l'alerte liée au diabète.

4. Dispositif selon l'une quelconque des revendications précédentes,
dans lequel le test de référence et le test d'auto-soin comprennent chacun un test cognitif pour détecter une capacité cognitive de l'utilisateur, dans lequel l'au moins un critère de réussite/échec comprend au moins une question, et dans lequel la réponse au test de référence et la réponse au test d'auto-soin comprennent chacune une indication d'au moins une réponse à l'au moins une question ;
dans lequel, éventuellement, le dispositif est en outre configuré pour fournir le test de référence et/ou le test d'auto-soin en superposant l'au moins une question sur un écran de verrouillage, un écran d'accueil ou une autre application affichée sur le dispositif mobile ;
dans lequel, éventuellement, l'au moins un critère de réussite/échec comprend un changement relatif d'accélération ou un changement relatif d'orientation du dispositif mobile sur une période de temps ;
dans lequel, éventuellement, la réponse au test de référence et la réponse au test d'auto-soin comprennent chacune une indication de l'accélération du dispositif mobile en se basant sur des données d'accéléromètre ou une indication de l'orientation du dispositif mobile en se basant sur des données de gyroscope ;
dans lequel, éventuellement, l'au moins un critère de réussite/échec prédéfini comprend un objet traçable ou un objet tactile sur un affichage du dispositif mobile, et dans lequel la réponse au test de référence et la réponse au test d'auto-soin comprennent chacune une indication d'au moins une entrée utilisateur relative à l'objet traçable ou à l'objet tactile sur l'affichage du dispositif mobile.

5. Dispositif selon l'une quelconque des revendications précédentes,
dans lequel l'état diabétique extrême comprend un événement hypoglycémique ou un événement hyperglycémique ; et/ou
dans lequel l'état diabétique extrême est détecté au niveau du seuil maximal ou du seuil minimal avant un seuil pour un événement hypoglycémique ou un événement hyperglycémique ; et/ou
dans lequel l'alerte liée au diabète est une alerte sonore communiquée par l'intermédiaire d'un haut-parleur du dispositif mobile.

6. Dispositif selon l'une quelconque des revendications précédentes, en outre configuré pour :
afficher une requête (308) pour communiquer l'alerte liée au diabète sur le dispositif mobile en réponse à la réussite du test d'auto-soin ; et
communiquer l'alerte liée au diabète en réponse à la réception d'une indication de l'utilisateur de communiquer l'alerte liée au diabète.

7. Dispositif selon l'une quelconque des revendications précédentes, en outre configuré pour :
démarrer un décompte (364) au niveau du dispositif mobile, et dans lequel le test d'auto-soin est fourni après la fin du décompte ;
dans lequel, éventuellement, le décompte correspond à un degré de sévérité de l'état diabétique extrême.

8. Dispositif selon l'une quelconque des revendications précédentes, en outre configuré pour :
démarrer un premier décompte au niveau du dispositif mobile ;
échouer à recevoir une indication que l'utilisateur a traité l'état diabétique extrême avant la fin du premier décompte ; et
démarrer un second décompte au niveau du dispositif mobile, dans lequel le test d'auto-soin est fourni après la fin du second décompte.

9. Dispositif configuré pour :
détecter (202), par l'intermédiaire d'une première information provenant d'un dispositif de surveillance de glucose sanguin, qu'un taux de glucose sanguin d'un utilisateur est entre un seuil maximal et un seuil minimal ;
après la détection du taux de glucose sanguin de l'utilisateur entre le seuil maximal et le seuil minimal, fournir (206) sur un dispositif mobile (104) un test de référence comprenant au moins un critère de réussite/échec prédéfini ;
établir une référence (212) associée à l'utilisateur en se basant sur la réponse au test de référence sur le dispositif mobile ;
détecter (304), par l'intermédiaire d'une seconde information provenant du dispositif de surveillance de glucose sanguin, un état diabétique extrême en réponse au fait que le taux de glucose sanguin de l'utilisateur est supérieur au seuil maximal ou inférieur au seuil minimal ;
en réponse à la détection de l'état diabétique extrême, fournir (312) sur le dispositif mobile un test d'auto-soin, dans lequel le test d'auto-soin comprend le même ou un sous-ensemble de l'au moins un critère de réussite/échec prédéfini ;
fournir un test supplémentaire pour confirmer les résultats du premier test en réponse à un échec du test d'auto-soin, dans lequel l'échec du test d'auto-soin est déterminé par un écart prédéfini entre la réponse au test de référence et la réponse au test d'auto-soin ;
communiquer (320) une alerte liée au diabète configurée pour solliciter une assistance dans le traitement de l'état diabétique extrême :
i) en réponse à la détection (316) de l'échec du test d'auto-soin et d'un échec du test supplémentaire ; et
ii) en l'absence (314) d'une réponse de l'utilisateur au test d'auto-soin dans une période de temps prédéfinie correspondant à un degré de sévérité et/ou au taux de glucose sanguin de l'utilisateur.

10. Dispositif selon la revendication 9, dans lequel le test d'auto-soin et le test de référence sont identiques.

11. Dispositif selon la revendication 9 ou la revendication 10,
dans lequel le test de référence et le test d'auto-soin comprennent chacun un test cognitif pour détecter une capacité cognitive de l'utilisateur, dans lequel l'au moins un critère de réussite/échec comprend au moins une question, et dans lequel la réponse au test de référence et la réponse au test d'auto-soin comprennent chacune une indication d'au moins une réponse à l'au moins une question ;
dans lequel, éventuellement, le dispositif est en outre configuré pour fournir le test de référence et/ou le test d'auto-soin en superposant l'au moins une question sur un écran de verrouillage, un écran d'accueil ou une autre application affichée sur le dispositif mobile ;
dans lequel, éventuellement, le dispositif est en outre configuré pour fournir le test supplémentaire en surveillant un changement relatif d'accélération du dispositif mobile associé à l'utilisateur sur une période de temps ou un changement relatif d'orientation du dispositif mobile sur une période de temps, et dans lequel l'échec du test supplémentaire est détecté en réponse au changement relatif d'accélération ou au changement relatif d'orientation dépassant un seuil de référence.

12. Dispositif selon l'une quelconque des revendications 9 à 11,
dans lequel le dispositif est en outre configuré pour fournir le test supplémentaire en affichant un objet traçable ou un objet tactile sur l'affichage du dispositif mobile, et dans lequel l'échec du test supplémentaire est détecté en se basant sur une indication d'au moins une entrée utilisateur relative à l'objet traçable ou à l'objet tactile sur l'affichage du dispositif mobile ; et/ou
dans lequel l'état diabétique extrême comprend un événement hypoglycémique ou un événement hyperglycémique ; et/ou
dans lequel l'état diabétique extrême est détecté au niveau du seuil maximal ou du seuil minimal avant un seuil pour un événement hypoglycémique ou un événement hyperglycémique ; et/ou
dans lequel l'alerte liée au diabète est une alerte sonore communiquée par l'intermédiaire d'un haut-parleur du dispositif mobile.

13. Dispositif selon l'une quelconque des revendications 9 à 12, en outre configuré pour :
afficher une requête (308) pour communiquer l'alerte liée au diabète sur le dispositif mobile en réponse à la réussite du test d'auto-soin ou du test supplémentaire ; et
communiquer l'alerte liée au diabète en réponse à la réception d'une indication de l'utilisateur de communiquer l'alerte liée au diabète.

14. Dispositif selon l'une quelconque des revendications précédentes, dans lequel le dispositif est en outre configuré pour communiquer l'alerte liée au diabète par au moins l'un des suivants :
envoi d'un premier message prédéfini pour l'alerte liée au diabète à un dispositif de haut-parleur externe pour communication sonore à un occupant d'un espace ;
envoi d'un second message prédéfini pour l'alerte liée au diabète par l'intermédiaire d'une télécommunication ou d'un message textuel à un dispositif alternatif au dispositif mobile ; ou
envoi d'un message d'alerte pour commander à un système d'automatisation domestique pour alerter l'utilisateur.

15. Dispositif selon la revendication 9,
dans lequel le test de référence comprend au moins un critère de réussite/échec prédéfini qui comprend un test cognitif comprenant au moins une question ;
dans lequel la référence indique une capacité cognitive de l'utilisateur ;
dans lequel la réponse au test de référence comprend une réponse à l'au moins une question du test cognitif ;
dans lequel le test supplémentaire comprend un test de dextérité fourni en affichant au moins un parmi un objet traçable ou un objet tactile sur le dispositif mobile et en détectant une indication d'au moins une entrée utilisateur relative à l'objet traçable ou à l'objet tactile sur l'affichage du dispositif mobile.
